# EUROPEAN PATENT APPLICATION

(11) **EP 4 632 015 A1**
(43) Date of publication of application: **15.10.2025**
(21) Application number: 24315138.8
(22) Date of filing: 09.04.2024
(51) Int. Cl.: C09B 57/00, A61B 5/00, A61K 49/00, C07F 5/02, C09K 11/06, G01N 33/533, C09B 23/04

(54) **COMPOUNDS FOR USE IN IN VIVO FLUORESCENCE-LIFETIME IMAGING**

(71) Applicant: Université de Bourgogne, 21000 Dijon (FR); Centre National de la Recherche Scientifique, 75016 Paris (FR)
(72) Inventor: Denat, Franck, 21800 QUETIGNY (FR); Chazeau, Elisa, 21000 DIJON (FR); Goze, Christine, 21560 Arc sur Tille (FR); Al Sabea, Hassan, 21300 Chenove (FR); Romieu, Anthony, 21490 Clenay (FR)
(74) Representative: Gevers & Orès

(57) **Abstract**

The present invention concerns compounds for use in *in vivo* fluorescence-lifetime imaging.
The compounds have the following formula (1):

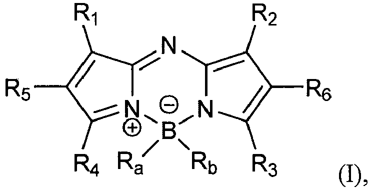

## Description

The present invention concerns compounds for use in *in vivo* fluorescence-lifetime imaging. Said invention also concerns new compounds, process of preparation and use thereof.

Among the methods for visualising and/or identifying biological processes or biological tissues *in vivo* using contrast agents, optical imaging has shown itself to be promising and attractive, with its relatively low cost, high sensitivity, simple instrumentation and use of non-ionising radiations. One of the major applications of this type of imaging is fluorescence guided surgery (FGS). Surgeons rely on visual inspection or palpation to determine the boundary between diseased tissue to be excised and healthy tissue, which does not always guarantee complete resection, thus increasing the risk of recurrence after tumour removal.

In this context, fluorescence imaging makes it possible to highlight cancer cells and provides intraoperative data enabling the surgeon to take the necessary margins for complete resection of the tumour mass.

However, traditional optical imaging has its limitations. Conventional systems detect the total fluorescence intensity emitted by a sample. But fluorescence intensity depends on the quantity of fluorophore accumulated, which is related to the size and/or thickness of the tumour, and detecting small lesions can thus be more complex.

Fluorescence intensity is also strongly affected by the absorption/scattering of light by the tissues and by autofluorescence generated by the tissues themselves.

A new optical imaging method, Fluorescence Lifetime Imaging (FLT), has emerged in this context, based not on the measurement of fluorescence intensity but on the measurement of the time that a fluorophore spends in the excited state (lifetime).

Fluorescence lifetime refers to the average time a fluorophore spends in its excited state before emitting a photon and returning to the ground state. FLT measures the decay of fluorescence over time, offering insights into the molecular environment and interactions of fluorophores. FLT uses time-correlated single photon counting (TCSPC) or other time-resolved techniques to capture the time delay between the excitation pulse and the emission of each photon. FLT also provides spatial information by analysing the fluorescence lifetime on a pixel-by-pixel basis, allowing the creation of lifetime maps.

Compared with fluorescence intensity, the fluorescence lifetime provides a stable measurement method which is less susceptible to artifacts arising from tissue absorption and scattering, excitation intensity variations, probes photobleaching, or unknown tissue penetration depth.

It means that many of the limitations mentioned above can possibly be overcome. Measuring the lifetime of a compound *in vivo* is indeed independent of the concentration of the fluorescent probe and of the intensity of the excitation light source, and is often a unique indicator of the local tissue environment. Lifetime imaging has better sensitivity and specificity for distinguishing fluorescent tracers from tissue autofluorescence and nonspecific tracer accumulation. With this method, the depth of penetration is also increased with a measurement that is less subject to light scattering/absorption phenomena. Lifetime imaging shows a significant improvement in contrast between the targeted tumour and the background noise compared with conventional optical imaging based on measurements of the intensity of the emitted light.

However, a barrier to the clinical development of this technology is the lack of fluorescent probes with both near infrared (NIR) emission, which is a prerequisite for *in vivo* use, and a lifetime greater than 1 ns in the biological environment. Cameras developed for lifetime imaging are indeed generally used with fluorophores already approved for clinical applications, which are characterised by good properties in terms of brightness in the NIR range, but lifetimes of less than a nanosecond. These short lifetimes make their detection complex, with a low differential compared with biological tissues. The development of fluorophores with lifetimes greater than 1.5 or 2 ns in a biological environment is therefore a prerequisite for the emerging use of this imaging method in clinical applications.

Until now, research has focused on the development of more powerful camera systems to measure the fluorescence lifetime of compounds in real time. Numerous devices, cameras and sensors have been developed for this application.

In sharp contrast, little research has been carried out to identify and use compounds that are better suited to fluorescence lifetime *in vivo.* As tissue autofluorescence is less marked above 650 nm, it is desirable to use fluorescent probes emitting in the NIR.

Proofs of concept for FLT imaging began with the study of compounds that do not emit in the NIR, such as GFP (emission centred around 510 mm), but have a relatively long lifetime (> 2 ns), making it possible to discriminate between emitted fluorescence and tissue autofluorescence.

However, the organic fluorophores currently used in the NIR, such as ICG or IRDye800CW, have a lifetime of less than 1 ns in a biological environment, which makes their detection complex.

There is a thus need for the development of new molecular tools for FLT.

Accordingly, it is an object of the present invention to provide NIR-emitting compounds aimed to be used in *in vivo* FLT, with lifetimes greater than 1, 1.5 or even 2, 3 or 4 ns. Such lifetimes, longer than those currently achieved by commercially available NIR fluorescent molecules, are particularly advantageous to facilitate their detection while injected *in vivo* for imaging.

Another aim of the invention is to provide compounds with good photophysical characteristics in the NIR (high quantum yield and molar absorption coefficient) under physiological conditions.

Another aim of the invention is to provide compounds with improved photostability compared with reference products (ICG, IRDye800CW).

Another aim of the invention is to provide compounds that can be easily modified, so that they can:
- have enhanced biocompatibility;
- be encapsulated within nanoparticles;
- exhibit a certain sensitivity in terms of fluorescence to different physiological conditions (pH, oxygen content, presence of particular enzymes, polarity and viscosity of certain cell organelles, etc.) and become so-called 'responsive' probes to changes in its environment; and/or
- be conjugated to a biological vector (antibodies, antibody fragments, peptide ligands, etc.) for specific targeting of tumours or other tissues *in vivo,* or to a chelator (synthesis of bimodal imaging agents using optical (FLT) and nuclear (PET and/or SPECT) modalities, or multimodal imaging agents using classical optical, FLT and radio-imaging modalities).

Thus, the present invention relates to a compound of following formula (I): 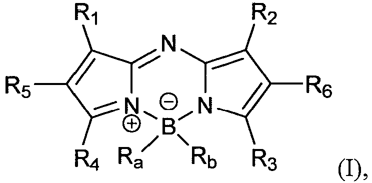 wherein:
R₁, R₂, R₃ and R₄, identical or different, represent a C₅-C₇ aryl or heteroaryl group, in particular a phenyl or a thiophenyl group, optionally substituted with at least one group chosen from halogen, nitro, -NR_{c}R_{d}, -OR_{d}, hydrazine, -CF₃, -CN, and groups constituted by or comprising a linker L and at least one group V,
R₅ and R₆, identical or different, represent a hydrogen, a halogen, a linear or branched (C₁-C₁₅)-group comprising an aldehyde, ketone, carboxylic acid or ester function, a nitrile, a sulfonate, a vinyl group optionally substituted by a ketone, ester or aromatic group, an imine substituted by an alkyl or aromatic group, an alkyne group optionally substituted by an alkyl or aromatic group, SPh, an aromatic chalcogen (SePh, TePh), an amide, a C₅-C₇ aryl or heteroaryl group, optionally substituted by at least one group chosen from a halogen, - NR_{c}R_{d}, -OR_{d}, hydrazine, -CF₃ and -CN,
optionally R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN,
R_{c} and R_{d}, identical or different, represent hydrogen or a linear or branched (C₁-C₅₀)-alkyl group, optionally interrupted by at least one atom or group chosen from -O-, -NR-, -S-, - C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, optionally substituted by at least one group comprising one or more heteroatoms chosen from O, N, P and/or S, in particular a group chosen from quaternary ammonium, sulphate, sulfonate, and phosphonate,
Rₐ and R_{b}, identical or different, represent:
   - a linear or branched (C₁-C₅₀)-alkyl group, optionally interrupted by at least one atom or group chosen from -O-, -NR-, -N⁺RR'-, -S-, -C(=O)-, -O-C(=O)-, -C(=O)-O-, - NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl,
   - a group constituted by or comprising a linker L and at least one group V,
V is chosen from:
   - groups comprising one or more heteroatoms chosen from O, N, P and/or S, in particular a group chosen from quaternary ammonium, sulphate, sulfonate, and phosphonate,
   - bioconjugable groups, in particular chosen from amine, ammonium, carboxylic acid, activated esters, in particular N-hydrosuccinimidyl, N-hydroxysulfosuccinimide, pentafluorophenyl, tetrafluorophenyl, squarate, in particular diethylsquarate, maleimide, thiol, isothiocyanate, isocyanate, oxadiazolyl methyl sulfone, azoture, optionally substituted tetrazine, triazole, alkyne, in particular terminal alkyne, trans-cyclooctene, cyclooctyne, in particular dibenzocyclooctyne and bicyclononyne,
   - a biological vector, in particular a cyclic or linear peptide, an antibody, an antibody fragment, a nanobody, an affibody, an aptamer, a short DNA or RNA sequence, a polysaccharide, a lipid, a sugar, an amino acid, a vitamin, a AMD3100 or AMD3100-type molecule, a prostate-specific membrane antigen (PSMA) ligand, a steroid, a fatty acid, a polyamine, a polyphenol, a DNA base or a caffeine derivative,
   - a nanoparticle, in particular a nanoparticle of 10 kDa or less or lipidic nanoparticles, more particularly a gold nanocluster or a lipid nanoparticle, for example a lipidot,
   - a metal complex, in particular for therapeutic purposes, formed by a chelating agent and a metal,
   - a radiometallic complex, formed by a chelating agent, for example DOTA (1,4,7,10-tetraazacyclododecane-1,4,7,10-tetraacetic acid), and a radiometal, in particular for therapeutic or diagnostic purposes,
   - a metal or radiometal chelating agent, for example DOTA,
   L is in particular a linear or branched (C₁-C₅₀)-alkyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, R and R', identical or different, represent hydrogen or a linear or branched (C₁-C₆)-alkyl group,
   or one of its tautomers,
   with the proviso that:
   - at least one of R₃ and R₄, in particular R₃ and R₄, represent(s) a C₅-C₇ heteroaryl group, in particular a thiophenyl group, optionally substituted with at least one group chosen from halogen, -NR_{c}R_{d}, -OR_{d}, hydrazine, -CF₃, -CN, and groups constituted by or comprising a linker L and at least one group V, with, in this case, R₁ and/or R₂ being different from *p*-N(Me)₂-phenyl, and optionally R^{a} and/or R^{d} being further different from -C≡C-CH₂-NH(CH₃)₂ and/or -C≡C-CH₂-N⁺(CH₃)₃, in particular different from -C≡C-CH₂-NH(CH₃)₂;
   - at least one of R^{a} and R^{d} represents a group V' of following formula -Lₐ-X-L_{b}-V, wherein Lₐ and L_{b}, identical or different, represent a linear or branched (C₁-C₅₀)-alkyl group, optionally interrupted by at least one atom or group chosen from -O-, - S-, -C(R)=C(R')-, -C=C-, X represents -NR- or -N⁺RR'-, and V is as defined above;
   - at least one of R₁, R₂, R₃ and R₄, in particular one of R₁, R₂, R₃ and R₄, represents a C₅-C₇ aryl or heteroaryl group, substituted with at least one group chosen from groups constituted by or comprising a linker L and at least one group V; and/or
   - R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and - CN.

In a particular embodiment, R₅ and/or R₆, in particular R₅ and R₆, represent a hydrogen.

In a particular embodiment, R₁ and/or R₂, in particular R₁ and R₂, represent a C₅-C₇ aryl, optionally substituted with at least one group chosen from halogen, nitro, -NR_{c}R_{d}, - OR_{d}, hydrazine, -CF₃, -CN, and groups constituted by or comprising a linker L and at least one group V.

In a particular embodiment, R₁ and/or R₂, in particular R₁ and R₂, represent a C₅-C₇ aryl, optionally substituted with at least one group chosen from halogen, nitro, -NR_{c}R_{d}, - OR_{d}, hydrazine, -CF₃, -CN.

In a particular embodiment, R₁ and/or R₂, in particular R₁ and R₂, represent a phenyl, optionally substituted with at least one group chosen from halogen, nitro, -NR_{c}R_{d}, -OR_{d}, hydrazine, -CF₃, and -CN.

In a particular embodiment, R₃ and/or R₄, in particular R₃ and R₄, represent a heteroaryl group, optionally substituted with at least one group chosen from halogen, nitro, -NR_{c}R_{d}, -OR_{d}, hydrazine, -CF₃, -CN, and groups constituted by or comprising a linker L and at least one group V.

In a particular embodiment, R₃ and/or R₄, in particular R₃ and R₄, represent a heteroaryl group, optionally substituted with at least one group chosen from halogen, nitro, -NR_{c}R_{d}, -OR_{d}, hydrazine, -CF₃, and -CN.

In a particular embodiment, R₃ and/or R₄, in particular R₃ and R₄, represent a heteroaryl group, optionally substituted with at least one group chosen from halogen, nitro, -NR_{c}R_{d}, -OR_{d}, hydrazine, -CF₃, and -CN, and R^{a} and/or R^{b}, in particular R^{a} and R^{b}, represent(s) -C≡C-CH₂-NRR' or -C≡C-CH₂-N⁺RR'R", with R, R' and R", identical or different, representing hydrogen or a linear or branched (C₁-C₆)-alkyl group.

In a particular embodiment, R₃ and/or R₄, in particular R₃ and R₄, represent a thiophenyl group, optionally substituted with at least one group chosen from halogen, nitro, -NR_{c}R_{d}, -OR_{d}, hydrazine, -CF₃, and -CN.

In a particular embodiment, R₃ and/or R₄, in particular R₃ and R₄, represent a thiophenyl group, optionally substituted with at least one group chosen from halogen, nitro, -NR_{c}R_{d}, -OR_{d}, hydrazine, -CF₃, and -CN, and R^{a} and/or R^{b}, in particular R^{a} and R^{b}, represent(s) -C≡C-CH₂-NRR' or -C≡C-CH₂-N⁺RR'R", with R, R' and R", identical or different, representing hydrogen or a linear or branched (C₁-C₆)-alkyl group.

In a particular embodiment, R₄ and R₅ and/or R₃ and R₆, in particular R₄ and R₅, and R₃ and R₆, are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN,
R₄ and R₅ and/or R₃ and R₆, in particular R₄ and R₅, and R₃ and R₆, forming for example a fluorene, optionally substituted by at least one group chosen from (C₁-C₆)-alkyl, halogen, - NR_{c}R_{d}, -OR_{d}, hydrazine, -CF₃ and -CN.

In a particular embodiment, R₄ and R₅ and/or R₃ and R₆, in particular R₄ and R₅, and R₃ and R₆, are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN, and R^{a} and/or R^{b}, in particular R^{a} and R^{b}, represent(s) -C≡C-CH₂-NRR' or -C≡C-CH₂-N⁺RR'R", with R, R' and R", identical or different, representing hydrogen or a linear or branched (C₁-C₆)-alkyl group,

R₄ and R₅ and/or R₃ and R₆, in particular R₄ and R₅, and R₃ and R₆, forming for example a fluorene, optionally substituted by at least one group chosen from (C₁-C₆)-alkyl, halogen, -NR_{c}R_{d}, -OR_{d}, hydrazine, -CF₃ and -CN. In a particular embodiment, R₁ and/or R₂, in particular R₁ or R₂, represent a C₅-C₇ aryl or heteroaryl group, substituted with at least one group Q constituted by or comprising a linker L and at least one group V.

In a particular embodiment, R₁ and/or R₂, in particular R₁ or R₂, represent a C₅-C₇ aryl, substituted with at least one group Q constituted by or comprising a linker L and at least one group V.

In a particular embodiment, R₁ and/or R₂, in particular R₁ or R₂, represent a phenyl, substituted with at least one group Q constituted by or comprising a linker L and at least one group V.

In a particular embodiment, R₃ and/or R₄, in particular R₃ or R₄, represent a C₅-C₇ aryl or heteroaryl group, substituted with at least one group Q constituted by or comprising a linker L and at least one group V.

In a particular embodiment, R₃ and/or R₄, in particular R₃ or R₄, represent a C₅-C₇ aryl, substituted with at least one group Q constituted by or comprising a linker L and at least one group V.

In a particular embodiment, R₃ and/or R₄, in particular R₃ or R₄, represent a phenyl, substituted with at least one group Q constituted by or comprising a linker L and at least one group V.

In a particular embodiment, Q is of formula -X-Lₐ-X'-L_{b}-V, wherein:
X and X' represent independently -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, or heteroarene diyl, with R and R', identical or different, representing hydrogen or a linear or branched (C₁-C₆)-alkyl group, X being for example -O-, and/or X' being for example -O-C(=O)-, -C(=O)-O-, -NR-C(=O)-, or -C(=O)-NR-,
Lₐ and L_{b}, identical or different, represent a linear or branched (C₁-C₅₀)-alkyl group, optionally interrupted by at least one atom or group chosen from -O-, -S-, -C(R)=C(R')-, - C=C-, Lₐ being for example a linear or branched (C₁-C₅₀)-alkyl group, and/or L_{b} being for example a linear or branched (C₁-C₅₀)-alkyl group, optionally interrupted by at least one atom -O-,
V is as defined above,
L being more particularly -O-CH₂-C(=O)-NH-L', with L' being a linear or branched (C₁-C₅₀)-alkyl group, optionally interrupted by at least one atom -O-.

In a particular embodiment, R₃ and/or R₄, in particular R₃ or R₄, represent a C₅-C₇ aryl or heteroaryl group, substituted with at least one group Q constituted by or comprising a linker L and at least one group V,
said group being in particular -X-Lₐ-X'-L_{b}-V, wherein:
X and X' represent independently -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, or heteroarene diyl, with R and R', identical or different, representing hydrogen or a linear or branched (C₁-C₆)-alkyl group, X being for example -O-, and/or X' being for example -O-C(=O)-, -C(=O)-O-, -NR-C(=O)-, or -C(=O)-NR-,
Lₐ and L_{b}, identical or different, represent a linear or branched (C₁-C₅₀)-alkyl group, optionally interrupted by at least one atom or group chosen from -O-, -S-, -C(R)=C(R')-, - C≡C-, Lₐ being for example a linear or branched (C₁-C₅₀)-alkyl group, and/or L_{b} being for example a linear or branched (C₁-C₅₀)-alkyl group, optionally interrupted by at least one atom -O-,
V is as defined above,
L being more particularly -O-CH₂-C(=O)-NH-L', with L' being a linear or branched (C₁-C₅₀)-alkyl group, optionally interrupted by at least one atom -O-,
and R^{a} and/or R^{d}, in particular R^{a} and R^{b}, represent(s) -C≡C-CH₂-NRR' or -C≡C-CH₂-N⁺RR'R", with R, R' and R", identical or different, representing hydrogen or a linear or branched (C₁-C₆)-alkyl group.

In a particular embodiment, R^{a} and/or R^{d}, in particular R^{a} and R^{b}, represent(s) -C= C-CH₂-NRR' or -C≡C-CH₂-N⁺RR'R", with R, R' and R", identical or different, representing hydrogen or a linear or branched (C₁-C₆)-alkyl group.

In a particular embodiment, at least one of R^{a} and R^{b}, in particular R^{a} or R^{a} and R^{b}, represent(s) a group constituted by or comprising a linker L and at least one group V.

In a particular embodiment, at least one of R^{a} and R^{d}, in particular R^{a} or R^{a} and R^{b}, represent(s) a group V' of following formula -Lₐ-X-L_{b}-V, wherein Lₐ and L_{b}, identical or different, represent a linear or branched (C₁-C₅₀)-alkyl group, optionally interrupted by at least one atom or group chosen from -O-, -S-, -C(R)=C(R')-, -C=C-, X represents -NR- or - N⁺RR'-, and V is as defined above.

In a more particular embodiment, Lₐ represents a linear or branched (C₁-C₅₀)-alkyl group, interrupted by a -C≡C- group.

In another more particular embodiment, L_{b} represents a linear or branched (C₁-C₅₀)-alkyl group, interrupted by at least one O atom, L_{b} representing in particular a PEG group.

In another more particular embodiment, Rₐ and/or R_{b}, in particular Rₐ and R_{b}, represent(s) a group of formula -C≡C-CH₂-NR-L_{b}-V or -C≡C-CH₂-N⁺RR'-L_{b}-V, wherein L_{b}, R, R', and V are as defined above, being in particular -C≡C-CH₂-NMe-L_{b}-V or -C≡C-CH₂-N⁺(Me)₂-L_{d}-V, L_{b} representing more particularly a linear or branched (C₁-C₅₀)-alkyl group or a PEG group.

In a particular embodiment:
- R₁ and/or R₂, in particular R₁ or R₂, represent a C₅-C₇ aryl or heteroaryl group, notably a C₅-C₇ aryl, for example a phenyl, substituted with at least one group constituted by or comprising a linker L and at least one group V;
- R₃ and/or R₄, in particular R₃ or R₄, represent a C₅-C₇ aryl or heteroaryl group, notably a C₅-C₇ aryl, for example a phenyl, substituted with at least one group constituted by or comprising a linker L and at least one group V; and/or, preferably or
- at least one of and R^{b}, in particular R^{a} or R^{a} and R^{d}, represent(s) a group constituted by or comprising a linker L and at least one group V, notably at least one of R^{a} and R^{b}, in particular R^{a} or R^{a} and R^{d}, representing a group V' of following formula -Lₐ-X-L_{b}-V, wherein Lₐ and L_{b}, identical or different, represent a linear or branched (C₁-C₅₀)-alkyl group, optionally interrupted by at least one atom or group chosen from - O-, -S-, -C(R)=C(R')-, -C=C-, X represents -NR- or -N⁺RR'-, and V is as defined above, with Lₐ representing for example a linear or branched (C₁-C₅₀)-alkyl group, interrupted by a -C=C- group, and/or L_{b} representing for example a linear or branched (C₁-C₅₀)-alkyl group, interrupted by at least one O atom, L_{b} representing for instance a PEG group.

In a particular embodiment, V is chosen from:
- groups comprising one or more heteroatoms chosen from O, N, P and/or S, in particular a group chosen from quaternary ammonium, sulphate, sulfonate, and phosphonate, in particular sulfonate,
- bioconjugable groups, in particular chosen from amine, ammonium, carboxylic acid, activated esters, in particular N-hydrosuccinimidyl, N-hydroxysulfosuccinimide, pentafluorophenyl, tetrafluorophenyl, squarate, in particular diethylsquarate, maleimide, thiol, isothiocyanate, isocyanate, oxadiazolyl methyl sulfone, azoture, optionally substituted tetrazine, triazole, alkyne, in particular terminal alkyne, trans-cyclooctene, cyclooctyne, in particular dibenzocyclooctyne and bicyclononyne, more particularly amine, ammonium, carboxylic acid, squarate, bicyclononyne,
- a metal or radiometal chelating agent, for example DOTA (1,4,7,10-tetraazacyclododecane-1,4,7,10-tetraacetic acid).

In a particular embodiment, the compound of the invention is chosen from: 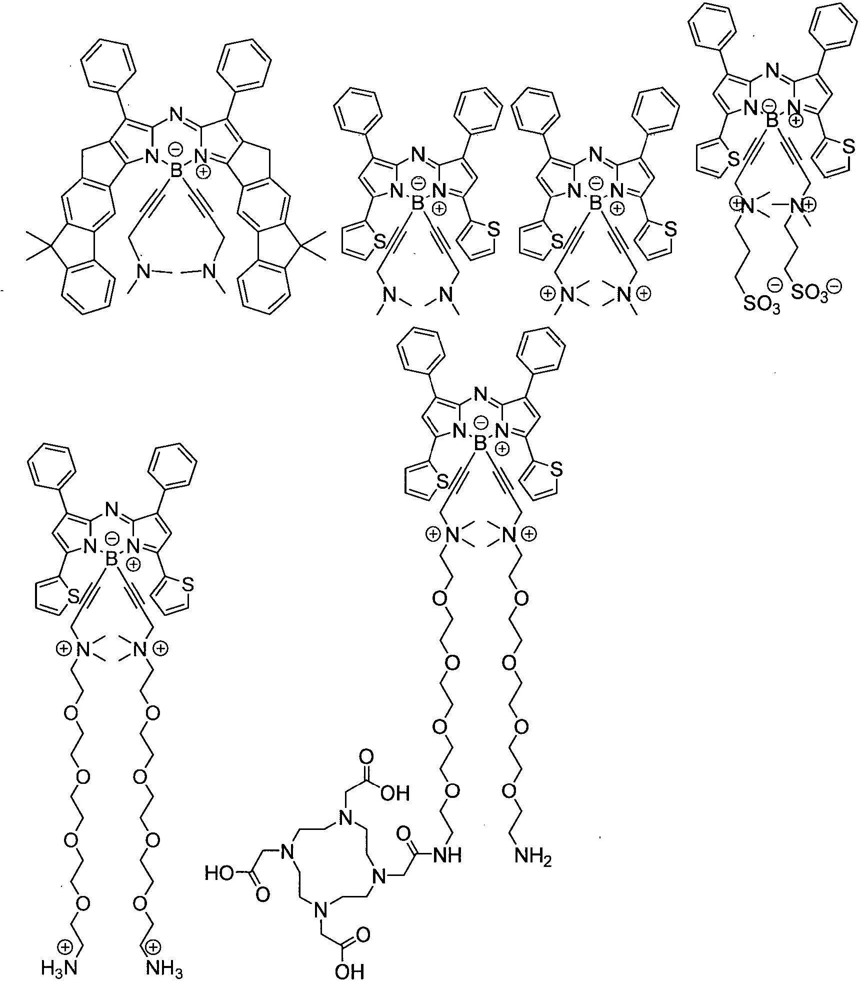 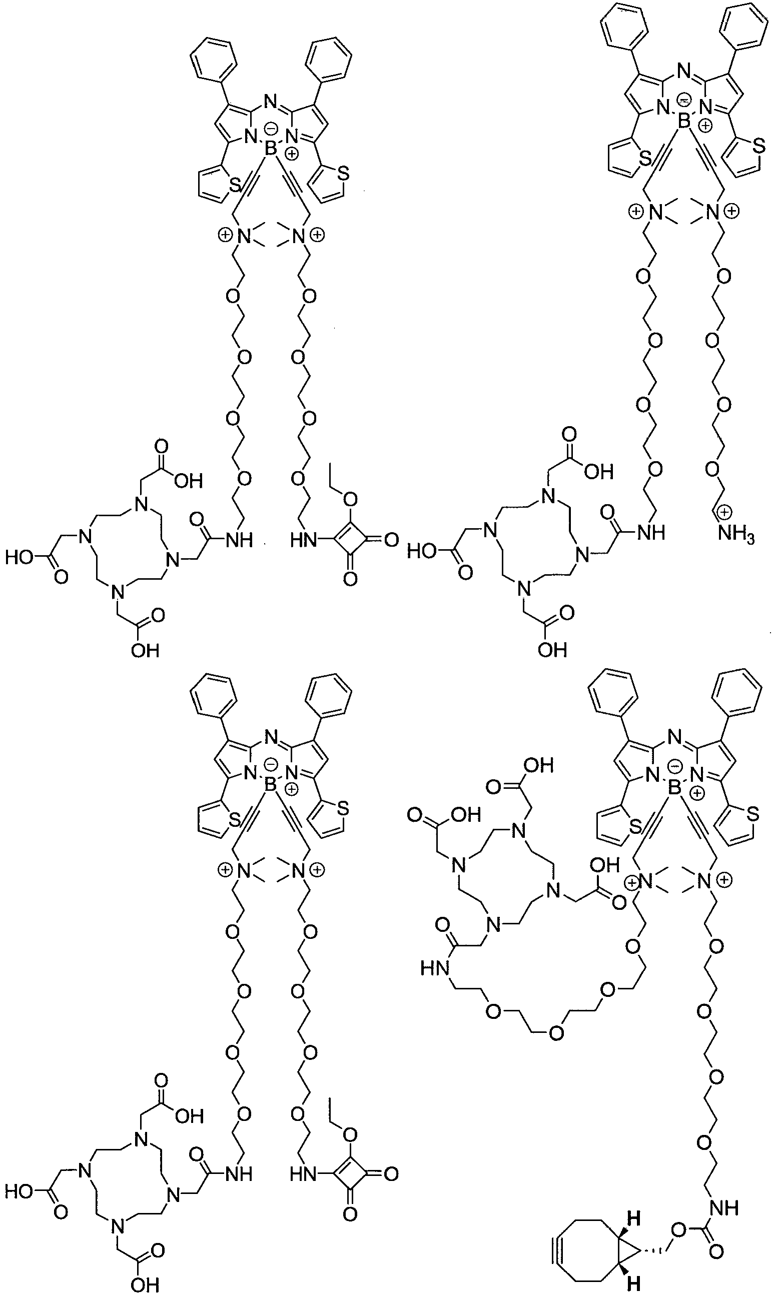 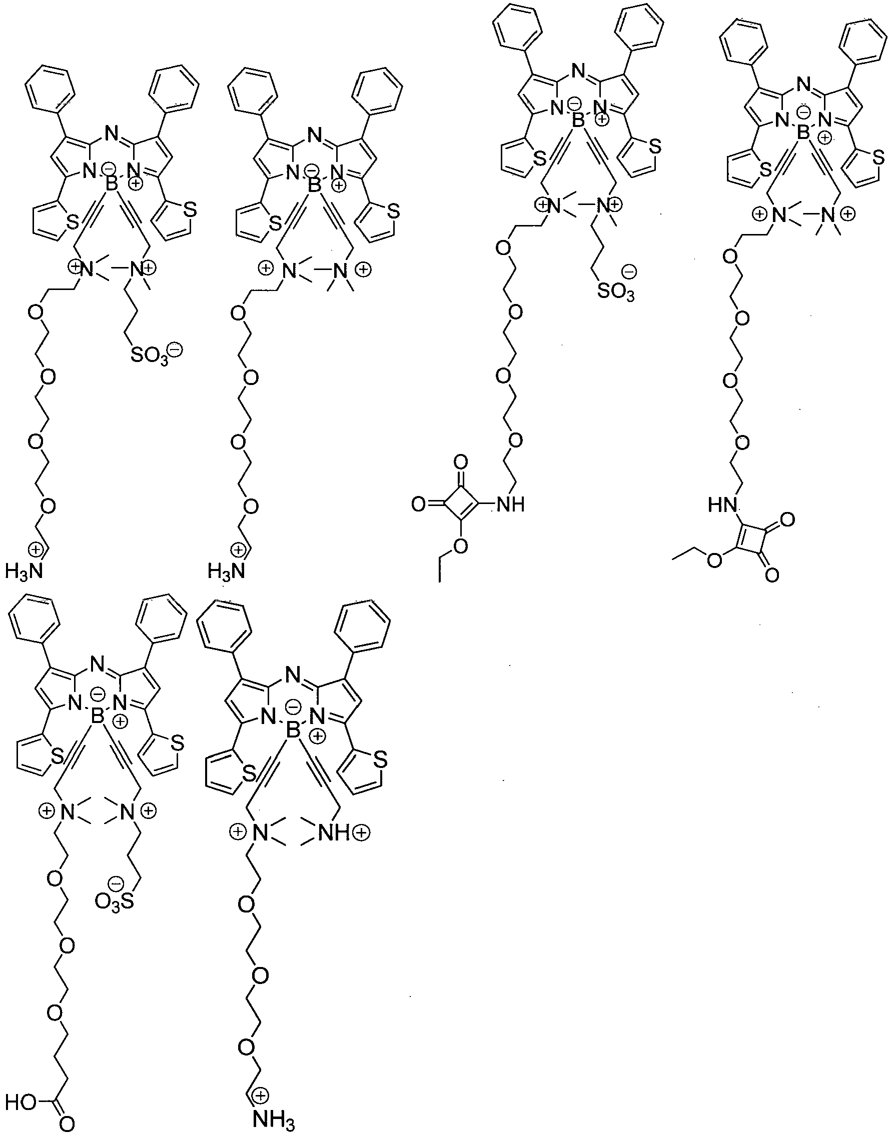 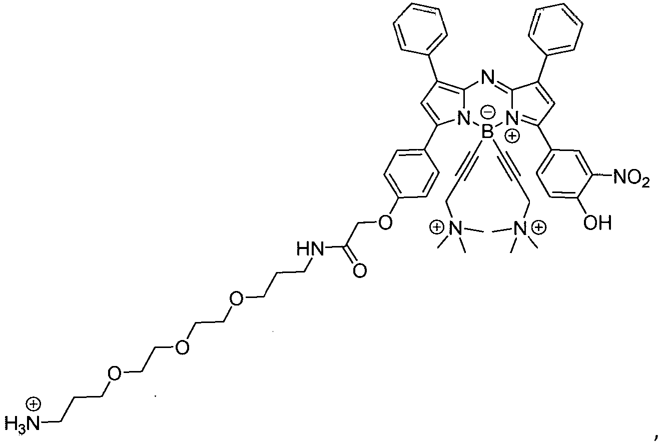 said compounds being optionally, if applicable, in the form of a formate or trifluoroacetate salt.

In another aspect, the present invention also concerns a compound of following formula (II), for use in a method of *in vivo* fluorescence-lifetime imaging: 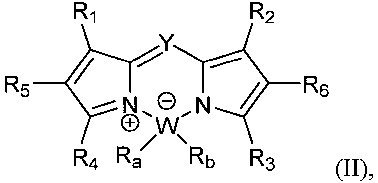 wherein:
W is chosen from B, PO₂, Ga, Al, In, Ru, Fe, Pt, Pd, Zr, Zn, Cu, Bi, Sb, Au, Rh, Ge, Sn, Ti;
W being in particular B;
Y is N or CZ, Y being in particular N;
Z is chosen from:
   - H;
   - Halogens;
   - OR" and SR", wherein R" represents hydrogen or a linear or branched (C₁-C₆)-alkyl group;
   - linear or branched (C₁-C₆)-alkyl group;
   - a C₅-C₇ aryl or heteroaryl group being optionally substituted with at least one group chosen from halogen, nitro, -NR_{c}R_{d}, -OR_{d}, hydrazine, -CF₃, -CN, and groups constituted by or comprising a linker L and at least one group V,
R₁, R₂, R₃ and R₄, identical or different, represent a linear or branched (C₁-C₆)-alkyl group, a C₅-C₇ aryl or heteroaryl group, a -CH₂=CH₂-(C₅-C₇) aryl or -CH₂=CH₂-(C₅-C₇) heteroaryl group, said aryl or heteroaryl group being optionally substituted with at least one group chosen from halogen, nitro, -NR_{c}R_{d}, -OR_{d}, hydrazine, -CF₃, -CN, and groups constituted by or comprising a linker L and at least one group V,
R₅ and R₆, identical or different, represent a hydrogen, a halogen, a linear or branched (C₁-C₁₅)-group comprising an aldehyde, ketone, carboxylic acid or ester function, a nitrile, a sulfonate, a vinyl group optionally substituted by a ketone, ester or aromatic group, an imine substituted by an alkyl or aromatic group, an alkyne group optionally substituted by an alkyl or aromatic group, SPh, an aromatic chalcogen (SePh, TePh), an amide, a C₅-C₇ aryl or heteroaryl group, optionally substituted by at least one group chosen from a halogen, - NR_{c}R_{d}, -OR_{d}, hydrazine, -CF₃ and -CN,
optionally R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN,
R_{c} and R_{d}, identical or different, represent hydrogen or a linear or branched (C₁-C₅₀)-alkyl group, optionally interrupted by at least one atom or group chosen from -O-, -NR-, -S-, -
C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C≡C-, arene diyl, in particular benzene diyl, and heteroarene diyl, optionally substituted by at least one group comprising one or more heteroatoms chosen from O, N, P and/or S, in particular a group chosen from quaternary ammonium, sulphate, sulfonate, and phosphonate,
Rₐ and R_{b}, identical or different, are absent or represent:
   - a halogen, in particular -F or -Cl,
   - a linear or branched (C₁-C₅₀)-alkyl group, optionally interrupted by at least one atom or group chosen from -O-, -NR-, -N⁺RR'-, -S-, -C(=O)-, -O-C(=O)-, -C(=O)-O-, - NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl,
   - a group constituted by or comprising a linker L and at least one group V,
R^{a} and R_{b} are absent when W is chosen from PO₂, Ga, Al, In, Ru, Fe, Pt, Pd, Zr, Zn, Cu, Bi, Sb, Au, Rh, Ge, Sn, Ti;
Rₐ and R_{b} are present when W is B;
V is chosen from:
   - groups comprising one or more heteroatoms chosen from O, N, P and/or S, in particular a group chosen from quaternary ammonium, sulphate, sulfonate, and phosphonate,
   - bioconjugable groups, in particular chosen from amine, ammonium, carboxylic acid, activated esters, in particular N-hydrosuccinimidyl, N-hydroxysulfosuccinimide, pentafluorophenyl, tetrafluorophenyl, squarate, in particular diethylsquarate, maleimide, thiol, isothiocyanate, isocyanate, oxadiazolyl methyl sulfone, azoture, optionally substituted tetrazine, triazole, alkyne, in particular terminal alkyne, trans-cyclooctene, cyclooctyne, in particular dibenzocyclooctyne and bicyclononyne,
   - a biological vector, in particular a cyclic or linear peptide, an antibody, an antibody fragment, a nanobody, an affibody, an aptamer, a short DNA or RNA sequence, a polysaccharide, a lipid, a sugar, an amino acid, a vitamin, a AMD3100 or AMD3100-type molecule, a prostate-specific membrane antigen (PSMA) ligand, a steroid, a fatty acid, a polyamine, a polyphenol, a DNA base or a caffeine derivative,
   - a nanoparticle, in particular a nanoparticle of 10 kDa or less or lipidic nanoparticles, more particularly a gold nanocluster or a lipid nanoparticle, for example a lipidot,
   - a metal complex, in particular for therapeutic purposes, formed by a chelating agent and a metal,
   - a radiometallic complex, formed by a chelating agent, for example DOTA (1,4,7,10-tetraazacyclododecane-1,4,7,10-tetraacetic acid), and a radiometal, in particular for therapeutic or diagnostic purposes,
   - a metal or radiometal chelating agent, for example DOTA,
L is in particular a linear or branched (C₁-C₅₀)-alkyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, R and R', identical or different, represent hydrogen or a linear or branched (C₁-C₆)-alkyl group,
or one of its tautomers.

In a particular embodiment, more particularly when R^{a} and R_{b} are absent, the coordination sphere of W may be completed with one or more ligands, for example a solvent molecule, notably chosen from pyridine, water, methanol, ethanol, tetrahydrofuran (THF).

In a particular embodiment, the present invention also concerns a compound of following formula (II₁), for use in a method of *in vivo* fluorescence-lifetime imaging: 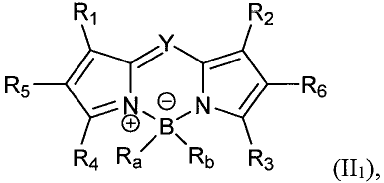 wherein:
Y is N or CH;
R₁, R₂, R₃ and R₄, identical or different, represent a C₅-C₇ aryl or heteroaryl group, optionally substituted with at least one group chosen from halogen, nitro, -NR_{c}R_{d}, -OR_{d}, hydrazine, - CF₃, -CN, and groups constituted by or comprising a linker L and at least one group V,
R₅ and R₆, identical or different, represent a hydrogen, a halogen, a linear or branched (C₁-C₁₅)-group comprising an aldehyde, ketone, carboxylic acid or ester function, a nitrile, a sulfonate, a vinyl group optionally substituted by a ketone, ester or aromatic group, an imine substituted by an alkyl or aromatic group, an alkyne group optionally substituted by an alkyl or aromatic group, SPh, an aromatic chalcogen (SePh, TePh), an amide, a C₅-C₇ aryl or heteroaryl group, optionally substituted by at least one group chosen from a halogen, - NR_{c}R_{d}, -OR_{d}, hydrazine, -CF₃ and -CN,
optionally R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN,
R_{c} and R_{d}, identical or different, represent hydrogen or a linear or branched (C₁-C₅₀)-alkyl group, optionally interrupted by at least one atom or group chosen from -O-, -NR-, -S-, -
C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, optionally substituted by at least one group comprising one or more heteroatoms chosen from O, N, P and/or S, in particular a group chosen from quaternary ammonium, sulphate, sulfonate, and phosphonate,
R^{a} and R_{b}, identical or different, represent:
   - a halogen, in particular -F or -Cl,
   - a linear or branched (C₁-C₅₀)-alkyl group, optionally interrupted by at least one atom or group chosen from -O-, -NR-, -N⁺RR'-, -S-, -C(=O)-, -O-C(=O)-, -C(=O)-O-, - NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl,
   - a group constituted by or comprising a linker L and at least one group V,
V is chosen from:
V is chosen from:
   - groups comprising one or more heteroatoms chosen from O, N, P and/or S, in particular a group chosen from quaternary ammonium, sulphate, sulfonate, and phosphonate,
   - bioconjugable groups, in particular chosen from amine, ammonium, carboxylic acid, activated esters, in particular N-hydrosuccinimidyl, N-hydroxysulfosuccinimide, pentafluorophenyl, tetrafluorophenyl, squarate, in particular diethylsquarate, maleimide, thiol, isothiocyanate, isocyanate, oxadiazolyl methyl sulfone, azoture, optionally substituted tetrazine, triazole, alkyne, in particular terminal alkyne, trans-cyclooctene, cyclooctyne, in particular dibenzocyclooctyne and bicyclononyne,
   - a biological vector, in particular a cyclic or linear peptide, an antibody, an antibody fragment, a nanobody, an affibody, an aptamer, a short DNA or RNA sequence, a polysaccharide, a lipid, a sugar, an amino acid, a vitamin, a AMD3100 or AMD3100-type molecule, a prostate-specific membrane antigen (PSMA) ligand, a steroid, a fatty acid, a polyamine, a polyphenol, a DNA base or a caffeine derivative,
   - a nanoparticle, in particular a nanoparticle of 10 kDa or less or lipidic nanoparticles, more particularly a gold nanocluster or a lipid nanoparticle, for example a lipidot,
   - a metal complex, in particular for therapeutic purposes, formed by a chelating agent and a metal,
   - a radiometallic complex, formed by a chelating agent, for example DOTA (1,4,7,10-tetraazacyclododecane-1,4,7,10-tetraacetic acid), and a radiometal, in particular for therapeutic or diagnostic purposes,
   - a metal or radiometal chelating agent, for example DOTA,
L is in particular a linear or branched (C₁-C₅₀)-alkyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, R and R', identical or different, represent hydrogen or a linear or branched (C₁-C₆)-alkyl group,
or one of its tautomers.

In a particular embodiment, said compound emits at a wavelength greater than or equal to 650 nm, in particular greater than or equal to 700 nm.

In a particular embodiment, said use includes a step of measuring fluorescence variations linked to variations in physiological conditions chosen from pH, oxygen content, presence of enzymes, polarity and viscosity of cell organelles.

For example, said use includes a step of measuring fluorescence variations linked to variations in pH, said compound comprising, notably as Z, R₁, R₂, R₃ or R₄, a phenol group, in particular a o-Cl-phenol, a o-nitro-phenol, or a*p*-N(CH₃)₂-phenyl.

For example, said use includes a step of measuring fluorescence variations linked to the presence of enzymes, said compound comprising, notably as Z, R₁, R₂, R₃ or R₄, a phenol group, in particular a*p*-nitro-OCH₃-phenyl.

For example, said use includes a step of measuring fluorescence variations linked to the presence of oxygen, said compound comprising, notably as Z, R₁, R₂, R₃ or R₄, a phenol group, in particular a*p*-N(Et)₂-phenyl.

For example, said use includes a step of measuring fluorescence variations in viscosity of cell organelles, said compound comprising, notably as Z, R₁, R₂, R₃ or R₄, a phenol group, in particular a (C₁-C₅₀)-alkyl-O-phenyl, in particular a *p*-(C₁-C₅₀)-alkyl-O-phenyl.

Said method of *in vivo* fluorescence-lifetime imaging may be used for fluorescence guided surgery (FGS).

Said method of *in vivo* fluorescence-lifetime imaging or fluorescence guided surgery may concern a patient, which is notably a mammal, in particular chosen from a human, for example a human child, a domestic livestock mammal, for example cattle (such as cows, beef, heifers and bulls), horses, donkeys, pigs, sheeps and goats, or a companion or research mammal, for example cats, horses, pigs, mice, rats, rabbits and ferrets, more particularly a dog or a mouse.

In a preferred embodiment, the patient is a human.

Said patient may be afflicted with one or more diseases and/or conditions that are notably cancer, endometriosis, atherosclerosis, diabetes or wound healing.

*In vivo* fluorescence-lifetime imaging is known from the skilled person in the art, as for example described by Peng, O. et al. (J. Fluorescence Lifetime Imaging of Cancer In Vivo. in In Vivo Fluorescence Imaging: Methods and Protocols (ed. Bai, M.) 55-66 (Springer, New York, NY, 2016) ; Smith, J. T. et al. (Optica 2022, 9 (5), pp. 532-544); or Kumar, A. T. et al. (Fluorescence lifetime-based contrast enhancement of indocyanine green-labeled tumors, JBO 22, 040501 (2017).All the embodiments defined above in relation with the compounds of formula (I) also apply here regarding compounds of formula (II), alone or in combinations.

In a particular embodiment, the compound of formula (II) is a compound of formula (I).

In a particular embodiment, said compound is chosen from:

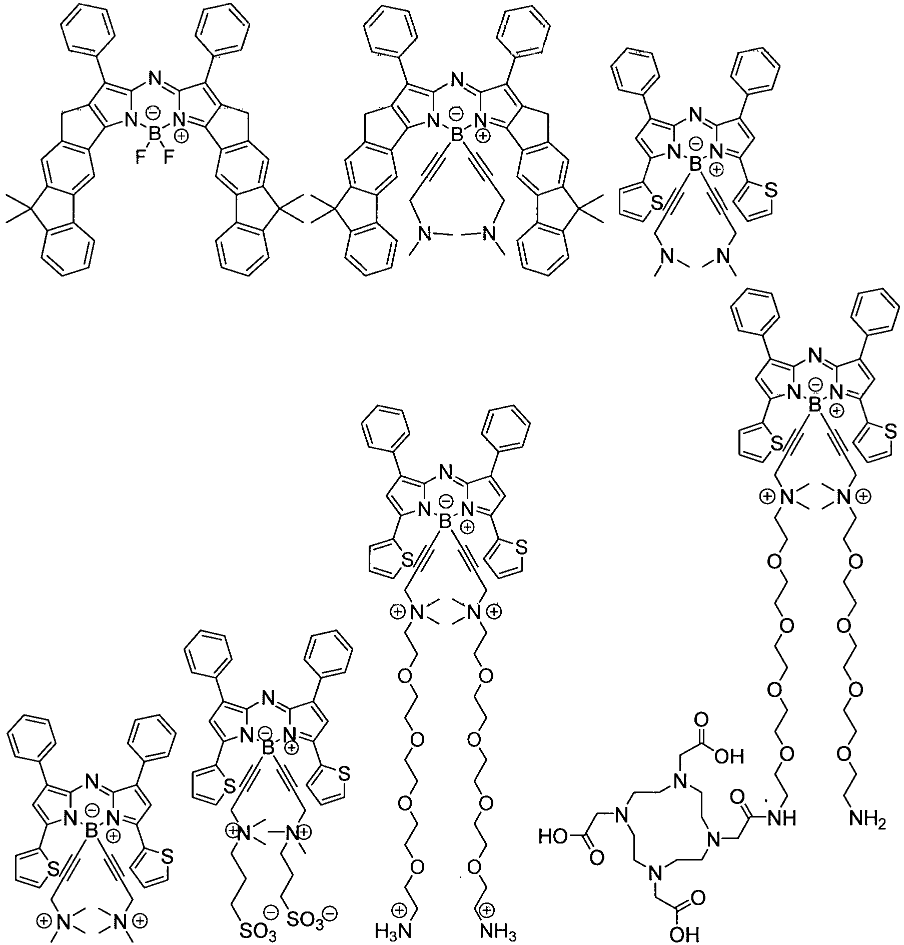

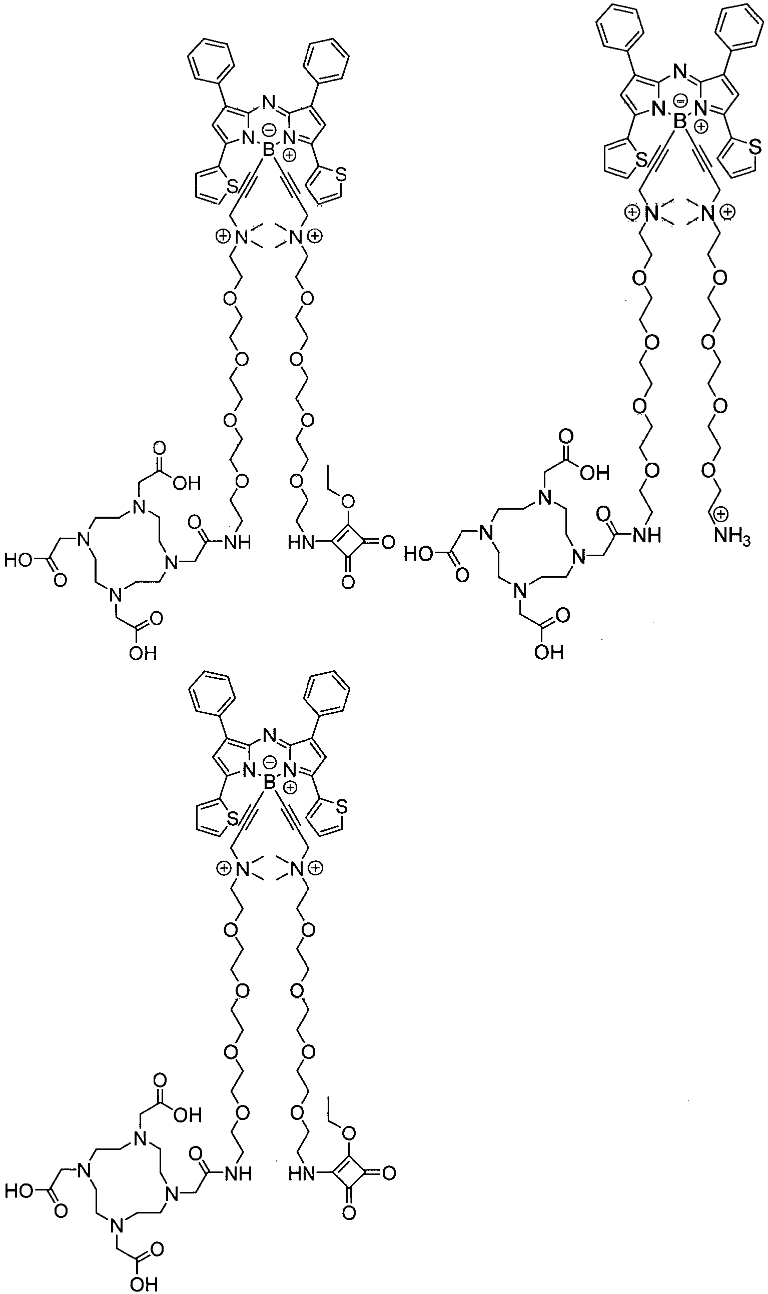

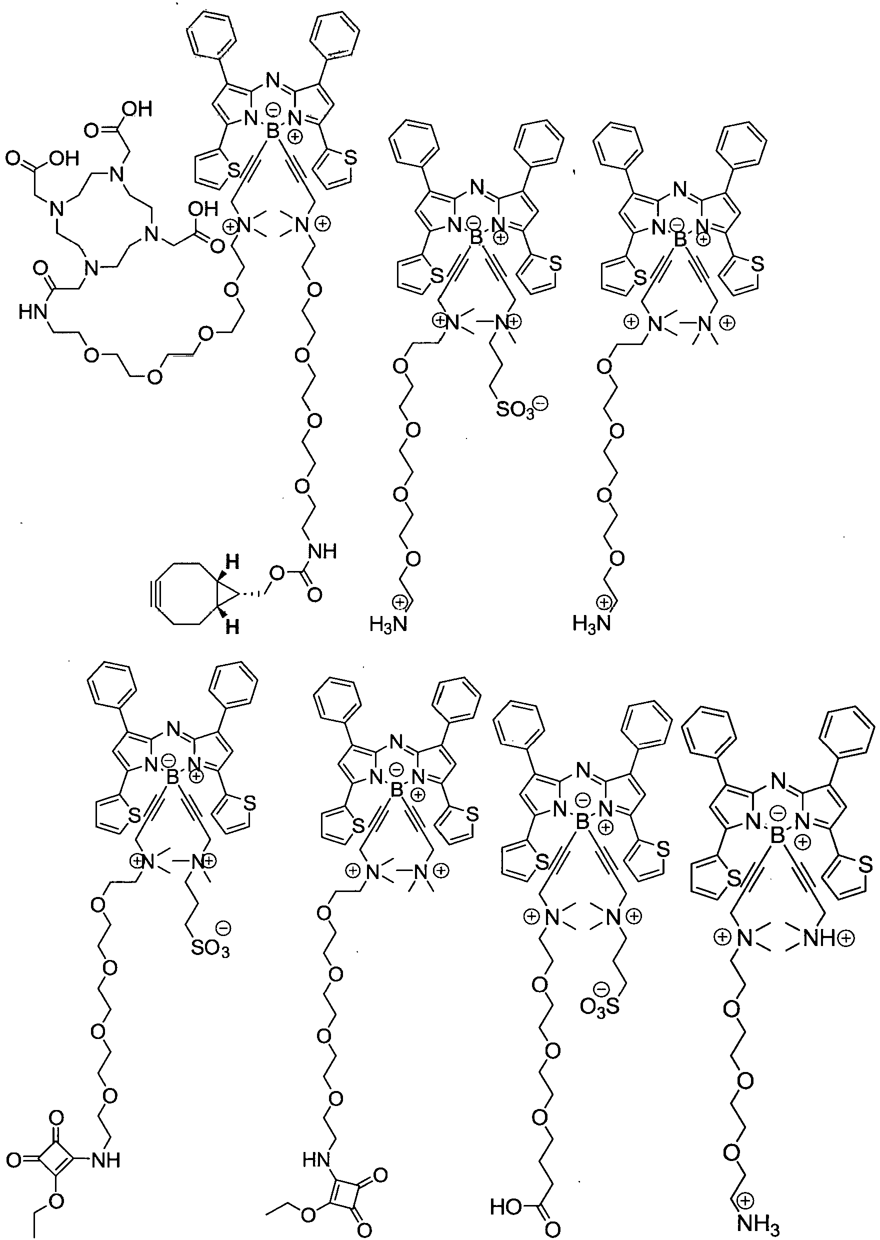

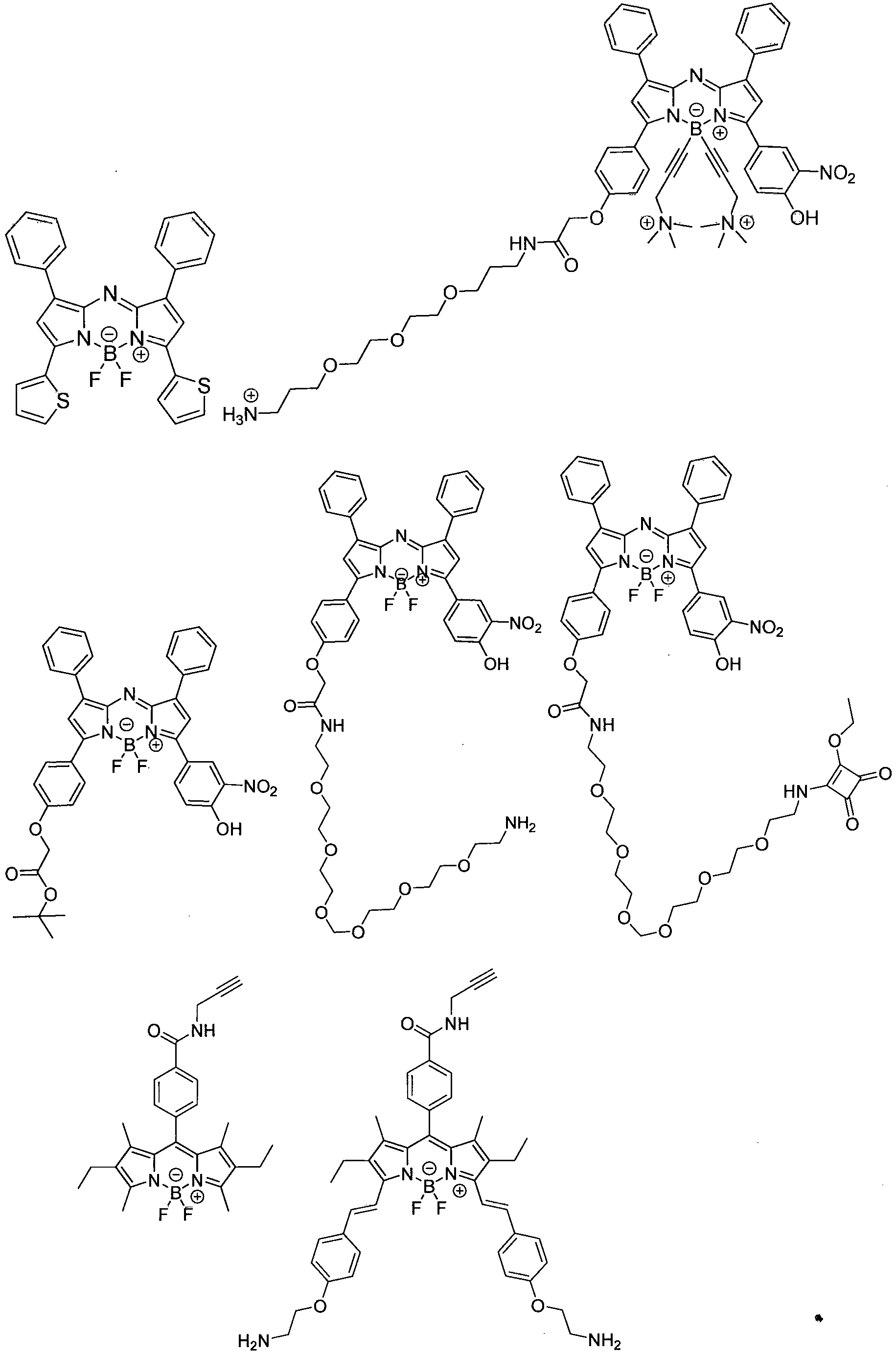

### DEFINITIONS

The following terms and expressions contained herein are defined as follows:

As used herein, a range of values in the form "x-y" or "x to y", or "x through y", include integers x, y, and the integers there between. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein, the term "patient" or "subject" refers to a warm blooded animal such as a mammal, notably a human, or a human child, which is in particular afflicted with, or has the potential to be afflicted with one or more diseases and conditions described herein.

The mammal may also be domestic livestock mammal, or a companion or research mammal.

Domestic livestock mammals include cattle (cows, beef, heifers and bulls), horses, donkeys, pigs, sheeps and goats.

Companion or research mammals include, without limitation thereto, dogs, cats, horses, pigs, mice, rats, rabbits and ferrets. Particularly contemplated companion or research mammals are a dog or a mouse.

By "diseases and conditions" are notably meant cancer, endometriosis, atherosclerosis, diabetes or wound healing.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as formic, acetic, benzenesulfonic, fumaric, glucoheptonic, gluconic, glutamic, glycolic, hydroxynaphthoic, 2-hydroxyethanesulfonic, lactic, maleic, malic, mandelic, methanesulfonic, muconic, 2-naphthalenesulfonic, propionic, succinic, dibenzoyl-L-tartaric, tartaric, p-toluenesulfonic, trimethylacetic, and trifluoroacetic acid and the like, the salts being for example formates or trifluoroacetates, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

It is also specified that all the formulae defined in the present specification include all the possible resonance structures, also called tautomers, being in particular noted that the structures represented herein may not be the most stable resonance structures.

The term "alkyl", as used in the present invention, refers in particular to a straight or branched saturated hydrocarbon chain, or a saturated or partially saturated mono- or bicyclic alkyl ring system.

Unless stated otherwise, the term "alkyl" thus includes the term "cycloalkyl", which refers in particular to a saturated or partially saturated mono- or bicyclic alkyl ring system.

The term "(C₁-C_{X})alkyl", as used in the present invention, refers in particular to a straight or branched saturated hydrocarbon chain containing from 1 to X carbon atoms.

For instance, the term "(C₁-C₇)alkyl", includes, but is not limited to, methyl, ethyl, n-propyl, iso-propyl, n-butyl, iso-butyl, sec-butyl, t-butyl, n-pentyl, n-hexyl, n-heptyl and the like, or a (C₃-C₇)cycloalkyl, such as cyclopropyl, cyclobutyl, cyclopentyl, cyclohexyl, cycloheptyl, cyclooctyl, pinenyl, adamantanyl and the like.

The term "alcoxy" as used in the present invention, refers in particular to alkyl-O-group, wherein the term "alkyl" is as defined above.

As used herein, the term "aryl" refers to a substituted or unsubstituted, mono- or bicyclic hydrocarbon aromatic ring system having 6 to 10 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

The term "halogen", as used in the present invention, refers in particular to a bromine, chlorine or iodine atom.

The term "alkenyl" refers in particular to any straight or branched hydrocarbon chain, carrying at least one carbon-carbon double bond, of 2 to 12 carbon atoms, preferably 2 to 6 carbon atoms, such as for example ethenyl, 2-propenyl, 2-butenyl, 3-butenyl, 2-pentenyl and its isomers, 2-hexenyl and its isomers, 2,4-pentadienyl.

The term "alkynyl" refers in particular to any linear or branched hydrocarbon chain, carrying at least one carbon-carbon triple bond, of 2 to 12 carbon atoms, preferably of 2 to 6 carbon atoms, such as for example ethynyl, 2-propynyl, 2-butynyl, 3-butynyl, 2-pentynyl and its isomers, 2-hexynyl and its isomers.

By "optionally interrupted", is in particular meant that the alkyl group comprises a given group, instead of a C-C bond, or in terminal position.

### EXAMPLES

### Example 1: synthesis of compounds according to the invention

### RP-HPLC separations

**System A:** semi-preparative RP-HPLC (SiliCycle SiliaChrom C18 column, 10 µm, 20×250 mm) with MeCN and aq. 0.1% TFA (pH 1.9) as eluents [10% MeCN (5 min), followed by a linear gradient from 10% to 100% MeCN (70 min)] at a flow rate of 20.0 mL/min. Quadruple UV-visible detection was achieved at 220, 260, 340 and 690 nm.

**System B:** semipreparative RP-HPLC (SiliCycle SiliaChrom C18 column, 10 µm, 20×250 mm) with MeCN and aq. 0.1% formic acid (FA, pH 2.1) as eluents [0% MeCN (5 min), followed by a linear gradient from 0% to 100% MeCN (70 min)] at a flow rate of 20.0 mL/min. Quadruple UV-visible detection was achieved at 220, 275, 640 and 670 nm.

**System C:** semipreparative RP-HPLC (Shimadzu Shimpack GIS C18 column, 5 µm, 10×250 mm) with MeCN and aq. 0.1% TFA (pH 1.9) as eluents [10% MeCN (3 min), followed by a linear gradient from 10% to 30% MeCN (4 min), followe by a linear gradient from 30% to 100% MeCN (40 min)] at a flow rate of 5.0 mL/min. Quadruple UV-visible detection was achieved at 220, 260, 340 and 690 nm.

**System D:** semi-preparative RP-HPLC (SiliCycle SiliaChrom C18 column, 10 µm, 20×250 mm) with MeCN and aq. 0.1% TFA (pH 1.9) as eluents [10% MeCN (5 min), followed by a linear gradient from 10% to 100% MeCN (70 min)] at a flow rate of 20.0 mL/min. Quadruple UV-visible detection was achieved at 220, 275, 640 and 670 nm.

**System E:** semipreparative RP-HPLC (Shimadzu Shimpack GIS C18 column, 5 µm, 10×250 mm) with MeCN and aq. 0.1% TFA (pH 1.9) as eluents [10% MeCN (3 min), followed by a linear gradient from 10% to 30% MeCN (4 min), followed by a linear gradient from 30% to 100% MeCN (40 min)] at a flow rate of 5.0 mL/min. Quadruple UV-visible detection was achieved at 220, 275, 640 and 670 nm.

### Synthesis of Aza-1:

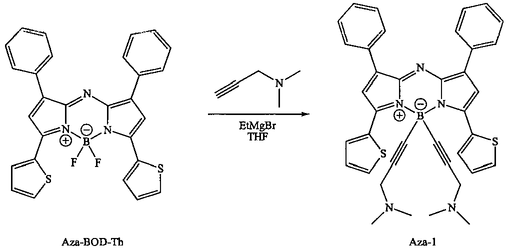

Aza-BOD-Th was synthesized according to a literature procedure (Tetrahedron, 2011; 67, 7148-7155).

In Schlenk tube fetched with a magnetic stirring bar, N,N-dimethylpropargylamine (160 µL, 0.148 mmol) was dissolved in dry THF (17 mL) and the solution was bubbled with argon gas for 5 minutes. A solution of ethylmagnesium bromide (1.81 mL, 0.9 M in THF, 1.63 mmol) was then added and the resulting reaction mixture was heated to reflux for 45 minutes. In a second Schlenk tube, Aza-BOD-Th (379 mg, 0.74 mmol) was dissolved in dry THF (17 mL). The mixture of the first Schlenk tube was transferred through a cannula to the second Schlenk tube. The final mixture was then refluxed for 45 minutes and the reaction was monitored by TLC, showing the complete consumption of the starting compound. The reaction was cooled and quenched by adding EtOH (3 mL). Thereafter, solvents were evaporated under reduced pressure and the resulting residue was purified by column chromatography (SiO₂, eluent = a step gradient of MeOH in DCM from 0% to 10%) to afford Aza-1 as glittery dark red-green powder (470 mg, yield 85%).

¹H NMR (500 MHz, CDCl₃): δ (ppm) = 8.83 (d, *J = 3.8 Hz,* 2H), 8.05 (d, *J* = *7.1 Hz,* 4H), 7.61(d, 2H, *J = 5.2 Hz),* 7.47-7.41 (multiplet, 6H), 7.27 (overlapping with residual signal of non-deuterated CHCl₃, d, 2H), 7.22 (s, 2H), 3.20 (s, 4H), 2.15 (s, 12H).

HRMS (ESI) (Da): m/z calcd. 318.6247 for [M + 2H]²⁺, found 318.62460.

### Synthesis of Waza-1 and Waza-2:

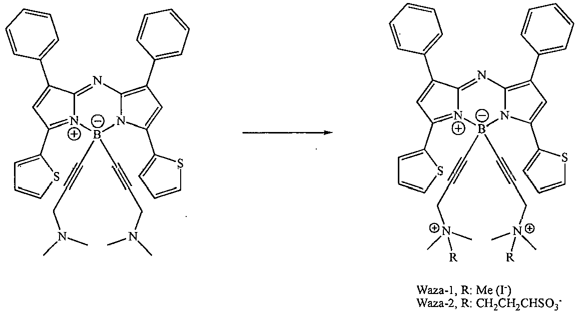

### Waza-1:

In a round bottom flask fetched with a magnetic stirring bar, Aza-1 (30 mg, 0.047 mmol) was dissolved in DCM (2 mL) followed by the addition of methyl iodide (MeI, 1 mL, 16 mmol, 340 equiv.). The resulting reaction mixture was stirred at room temperature for 30 minutes. Then, volatiles (DCM and MeI) were evaporated under reduced pressure. Waza-1 was obtained in a quantitative yield (43 mg).

¹H NMR (500 MHz, CD₃OD): δ (ppm) = 8.67 (d, *J = 3.8 Hz,* 2H), 8.12 (d, *J* = *7.1 Hz,* 4H), 8.00 (d, *J = 5.2 Hz,* 2H), 7.57 (s, 2H), 7.52-7.44 (m, 6H), 7.45 (t, *J = 4.6 Hz,* 2H), 4.17 (s, 4H), 2.94 (s, 18H).

HRMS (ESI) (Da): m/z calcd. 332.64036 for [M-2I]²⁺, found 332.64032.

### Waza-2:

In a round bottom flask fetched with a magnetic stirring bar, Aza-1 (30 mg, 0.047 mmol) was dissolved in MeCN (2 mL) followed by the addition of 1,3-propanesultone (12 mg, 0.1 mmol, 21 equiv.) and anhydrous K₂CO₃ (25 mg, 0.19 mmol, 4 equiv.). The resulting reaction mixture was stirred at 50 °C for 4 hours. The reaction was monitored by LC-MS showing the formation of Waza-2 as the major product. Thereafter, the mixture was filtered to remove inorganic salts, and directly purified by semi-preparative RP-HPLC (System A, Rt= 33 - 35 mins). The product containing fractions were lyophilized to give TFA salt of Waza-2 as a dark green amorphous powder (18 mg, yield 40% based on TFA mass = 20% determined by ion chromatography).

¹H NMR (500 MHz, CD₃OD): δ (ppm) = 8.71 (d, *J = 3.8 Hz,* 2H), 8.11 (d, *J = 7.2 Hz,* 4H), 8.01 (d, *J = 5.1 Hz,* 2H), 7.56 (s, 2H), 7.52-7.46 (m, 8H), 4.10 (s, 4H), 3.43 (m, 4H), 2.91(s, 12H), 2.78 (t, *J* = *6.2 Hz,* 4H), 2.11 (m).

HRMS (ESI) (Da): m/z calcd. 462.61042 for [M + 2Na]²⁺, found 462.60993

### Waza-BC1:

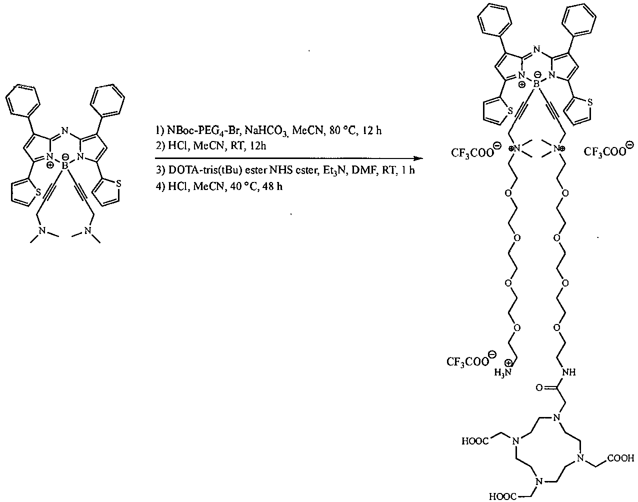

Steps 1 (N-alkylation) and 2 (Boc removal):
In a round bottom flask fetched with a magnetic stirring bar, Aza-1 (100 mg, 0.15 mmol) was dissolved in MeCN (10 mL) followed by the addition of N-Boc-PEG₄-bromide (purchased from BroadPharm company, 190 mg, 0.47 mmol, 3.1 equiv.) and NaHCO₃ (66 mg, 0.78 mmol, 5.2 equiv.). The reaction mixture was heated under refluxed overnight. The reaction was monitored by LC-MS showing the complete consumption of starting Aza-1 and formation of the bis-PEGylated product. Then, the crude mixture was filtered to remove inorganic salts and concentrated under reduced pressure. The resulting residue was dissolved in MeCN (3 mL) and aq. 1.0 M HCl was added (1 mL, 6.7 equiv.). The mixture was left under stirring at room temperature overnight. LC-MS analysis showed the formation of the mono-deprotected derivative as the major product with the full-deprotected product as the side product. The mono N-Boc derivative was purified by semi-preparative RP-HPLC (System A, Rₜ = 33 - 35 mins). The product containing fractions were lyophilized to give TFA salt of mono-amine intermediate which was used directly in the next step (80 mg, yield 44%).

Steps 3 (N-acylation with DOTA-tris(tBu) ester NHS ester followed by Boc removal) and 4 (introduction of reactive handle for bioconjugation):
TFA salt of mono-amine (40 mg, 0.041 mmol) was dissolved in dry DMF (2 mL) followed by the addition of DOTA-tris(tBu) ester NHS ester (provided by CheMatech company, 42 mg, 0.051 mmol, 1.2 equiv.) and triethylamine (TEA, 24 µL, 0.17 mmol, 5 equiv.). The resulting reaction mixture was stirred at 40 °C for 1 hour to reach completion (confirmed by LC-MS analyses). Thereafter, volatiles were evaporated under reduced pressure. The crude residue was redissolved in MeCN (2 mL) and aq. 4.0 M HCl (1 mL, 117 equiv.) was added.
The reaction mixture was stirred at 40 °C for 48 hours. LC-MS analysis showed the only formation of Waza-BC1. This later one was purified by semi-preparative RP-HPLC (System A, Rₜ = 25 - 27 mins). The product containing fractions were lyophilized to give TFA salt of Waza-BC1 as a dark blue spongy solid (32 mg, yield 34% based on TFA mass = 38% determined by ion chromatography).

¹H NMR (500 MHz, CD₃OD): δ (ppm) 8.66 = (d, *J = 3.9 Hz,* 2H), 8.14 (d, *J* = *7.1 Hz,* 4H), 8.02 (d, *J = 5.2 Hz,* 2H), 7.62 (s, 2H), 7.54-7.48 (m, 6H), 7.43 (t, *J* = *4.7 Hz,* 2H), 4.22 (s, 2H), 4.21(s, 2H), 3.81(broad peak, 5H), 3.73 (broad peak, 5H), 3.59 (t, *J* = *4.7 Hz,* 3H), 3.53-3.33 (m, 40H), 3.25 (2 broad peaks, 8H), 3.04 (t, 3H, *Jc= 4.6 Hz),* 2.95 (broad singlet, 12H).

HRMS (ESI) (Da): m/z calcd. 487.57699 for [M-3TFA]³⁺, found 487.55702.

### Waza-BC1-Sq:

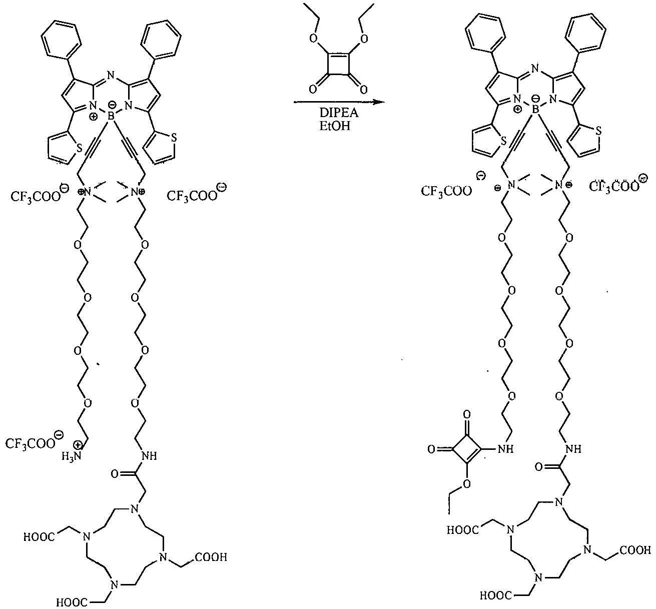

In a round bottom flask fetched with a magnetic stirring bar, Waza-BC1 (15.4 mg, 10 µmol) was dissolved in absolute EtOH (2 mL) followed by the addition of diethyl squarate (17.86 µL, 91 µmol, 9.1 equiv.) and N,N-diisopropylethylamine (DIPEA, 17.85 µL, 105 µmol, 10.5 equiv.). The reaction mixture was stirred at 35 °C for 1 hour. LC-MS analysis showed the complete consumption of the starting amine and the only formation of the desired product. Volatiles were then evaporated under reduced pressure. The resulting residue was purified by semi-preparative RP-HPLC (System C, Rₜ = 17 - 19 mins), The product containing fractions were lyophilized to give TFA salt of Waza-BC1-Sq as a dark blue sponge (10 mg, yield 62%, calculated without counting TFA counterions).

HRMS (ESI) (Da): m/z calcd. 528.91567 for [M+H-2TFA]³⁺, found 528.91553.

### Synthesis of Aza-2, Aza-3, Aza-4, and Waza-pH-1

Aza-OMe was prepared according to a literature procedure. (Bioconjugate Chem. 2019; 30, 1061-1066).

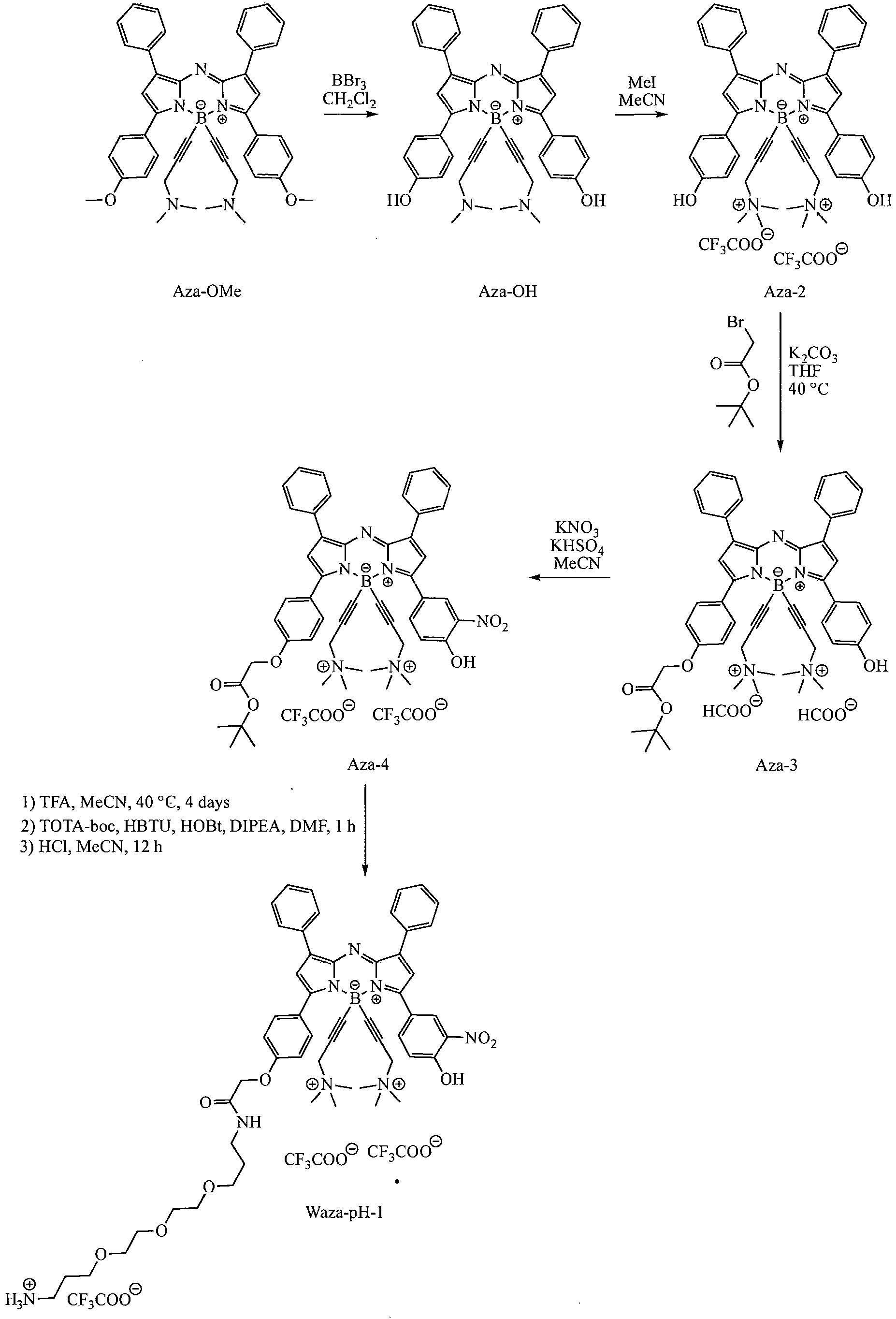

### Aza-OH:

In a round bottom flask fetched with a magnetic stirring bar, Aza-OMe (0.5 g, 0.7 mmol) was dissolved in dry DCM (100 mL) followed by the slow addition of a solution of boron tribromide (3.65 mL, 3.65 mmol, 1.0 M in DCM). The reaction mixture was stirred at room temperature overnight. A further amount of boron tribromide solution in dichloromethane (3.65 mL) was added to reach reaction completion (monitored by LC-MS analyses).

Thereafter, the reaction mixture was quenched by adding methanol (5 mL) and stirring for 30 minutes. Solvents were then evaporated under reduced pressure. The resulting residue was purified by column chromatography (SiO₂, eluent = DCM/MeOH 90:10, v/v) to afford Aza-OH as a dark blue glittery powder (220 mg, yield 45%).

¹H NMR (500 MHz, CD₃OD) δ (ppm): 8.29 (d, *³J = 8. 7 Hz,* 4H), 8.11 (d, *³J* = *8.1 Hz,* 4H), 7.50-7.44 (m, 6H), 7.31 (s, 2H), 6. 98 (d, 4H, *³J= 8. 7 Hz),* 3.75 (s, 4H), 2.58 (s, 18H). HRMS (ESI) (Da): m/z calcd. 326.66320 for [M + 2H]²⁺, found 326.66315.

### Aza-2:

In a round bottom flask fetched with a magnetic stirring bar, Aza-OH (83 mg, 0.126 mmol) was dissolved in MeCN (10 mL) followed by the addition of MeI (5 mL) and NaHCO₃ (160 mg, 1.9 mmol, 15 equiv.). The reaction mixture was stirred for at room temperature overnight. LC-MS analysis showed the complete consumption of starting Aza-OH and the complete formation of Aza-2. The mixture was then filtrated to remove inorganic salts and evaporated under reduced pressure. The resulting residue was purified by semi-preparative RP-HPLC (System D, Rₜ = 23 - 27 mins) to afford TFA salt of Aza-2 (95 mg, 82% yield, calculated assuming each Aza-2 contains 2 TFA counterions).

¹H NMR (500 MHz, CD₃OD): δ (ppm) = 8.29 (d, *³J = 8.8 Hz,* 4H), 8.11 (d, *³J = 8.1 Hz,* 4H), 7.49-7.41 (m, 6H), 7.34 (s, 2H), 6.92 (d, *³J = 8.8 Hz,* 4H), 4.01 (s, 4H), 2.89 (s, 18H). HRMS (ESI) (Da): m/z calcd. 342.67885 for [M-2TFA]²⁺, found 342.67871.

### Aza-3:

In a round bottom flask fetched with a magnetic stirring bar, Aza-2 (95 mg, 0.1 mmol) was dissolved in MeCN (10 mL) followed by the addition of anhydrous K₂CO₃ (138.2 mg, 1 mmol, 10 equiv.). The reaction mixture was stirred 5 minutes before adding *tert*-butyl bromoacetate (8 µL, 0.052 mmol, 0.5 equiv.). The reaction was left stirring at 40 °C overnight. LC-MS analysis showed the formation of the desired mono-O-alkylated product as the major product. Then, the mixture was filtered to remove inorganic salts and concentrated under reduced pressure. The resulting residue was purified by semi-preparative RP-HPLC (System D, R_{f} = 32-37 mins, System B, Rₜ = 27 - 30 mins) to give TFA salt of Aza-3 as a dark green solid (45 mg, yield 48%, based on HCOOH mass = 6.25 % determined by ion chromatography). *Please note:* the minor product was the starting bis-phenolic compound which was recovered during the RP-HPLC purification step to be reused for further synthesis of Aza-3. Only product purified RP-HPLC (System B) works in the next step.

¹H NMR (500 MHz, DMSO-*d*₆): δ (ppm) = 8.34 (d, *J* = *8.7 Hz,* 2H), 8.30 (d, *J* = *8.8 Hz,* 2H), 8.15 (t, *J = 6.5 Hz,* 4H), 7.65 (s, 2H), 7.56-7.45 (m, 6H), 7.13 (d, *J = 8.9 Hz,* 2H), 6.99 (d, *J = 8.8 Hz,* 2H), 4.83 (s, 2H), 4.03 (s, 4H), 2.80 (s, 18H), 1.48 (s, 9H).

HRMS (ESI) (Da): m/z calcd. 399.71289 for [M-2HCOOH]²⁺, found 399.71275.

### Aza 4:

In a round bottom flask fetched with magnetic stirring bar, Aza-3 (45 mg, 0.043 mmol) was dissolved in MeCN (4 mL) and the resulting solution heated at 85 °C. In parallel, KNO₃ (4.85 mg, 0.048 mmol, 1.1 equiv.) and KHSO₄ (8.85 mg, 0.043 mmol, 1 equiv.) was dissolved in ultrapure water (0.4 mL) and added slowly to the warm mixture. After 10 minutes, the solution turned blue. The reaction was immediately quenched by cooling it down to room temperature. LC-MS analysis showed the formation of the desired *ortho-*nitro product with some impurities. The reaction mixture was directly purified by semi-preparative RP-HPLC (System D, Rₜ = 27 - 30 mins) to give TFA salt of Aza-4 as a dark green solid (15 mg, yield 32%, based on TFA mass = 20 % determined by ion chromatography).

¹H NMR (500 MHz, DMSO-*d*₆): δ (ppm) = 9.15 (d, ⁴*J = 2.2 Hz,* 1H), 8.44 (d, *J = 8.9 Hz,* 2H), 8.35 (dd, *²J = 9.1 Hz, ³J = 2.3 Hz,* 1H), 8.17 (d, *J* = *7.01 Hz,* 2H), 8.14 (d, *J* = *7.01 Hz,* 2H), 7.71 (s, 1H), 7.67 (s, 1H), 7.56-7.47 (m, 6H), 7.27 (d, *J = 9.1 Hz,* 1H), 7.18 (d, *J = 9.0 Hz,* 2H), 4.87 (s, 2H), 4.04 (s, 4H), 2.82 (s, 18H), 1.48 (s, 9H).

HRMS (ESI) (Da): m/z calcd. 422.20543 for [M-2TFA]²⁺, found 422.20634.

### Waza-pH-1:

### Step 1

In a round bottom flask fetched with a magnetic stirring bar, Aza-4 (15 mg, 0.014 mmol) was dissolved in MeCN (3 mL) followed by the addition of TFA (0.2 mL). The reaction mixture was stirred at 40 °C until complete deprotection of Aza-4 (complete *tert*-butyl ester removal checked by LC-MS analyses). Thereafter, the reaction mixture was co-evaporated several times with DCM to completely remove traces of TFA. The resulting free carboxylic acid derivative was used in the next step without further purification.

### Step 2

This compound was then dissolved in dry DMF followed by the addition of (12 mg, 0.088 mmol, 6.2 equiv.), HBTU (33 mg, 0.088 mmol, 6.2 equiv.), and DIPEA (14.8 µL, 0.088 mmol, 6.2 equiv.) and left stirring for 30 minutes. Then, Boc-TOTA (28 mg, 0.088 mmol, 6.2 equiv., purchased from Iris Biotech GmbH) was added and the mixture was stirred at room temperature for 1 hour. The reaction was checked for completion by LC-MS and the mixture was concentrated under reduced pressure. The coupling product was used in the next step without further purification.

### Step 3

This N-Boc derivative was dissolved in MeCN (1 mL) and aq. 1.0 M HCl (1 mL) was added. The reaction mixture was stirred at room temperature overnight. The reaction was checked for completion by LC-MS anlaysis. The reaction mixture was directly purified by semi-preparative RP-HPLC (System E, Rₜ = 14 - 16 mins) to give TFA salt of Aza-4 as a dark green solid (10 mg, yield 63% based on TFA mass = 16.57 % determined by ion chromatography).

¹H NMR (500 MHz, CD₃OD): δ (ppm) = 9.35 (d, *⁴J* = 2.2 *Hz,* 1H), 8.44 (d, *J = 8.9 Hz,* 2H), 8.28 (dd, *²J = 9.1 Hz, ³J = 2.3 Hz,* 1H), 8.13 (multiplet, 2H), 7.53-7.47 (multiplet + singlet, 7H), 7.38 (s, 1H), 7.33 (d, *J = 9.1 Hz,* 1H), 7.22 (d, *J = 9.0 Hz* 2H), 4.72 (s, 2H), 4.04 (s, 4H), 3.67-3.60 (multiplet, 10H), 3.55 (t, *J =* 6. 7 *Hz,* 2H), 3.38 (t, *J = 6.3 Hz,* 2H), 3.09 (t, *J = 6. 6 Hz,* 2H) , 2.91 (s, 18H), 1.92 (multiplet, 2H), 1.84 (multiplet, 2H).

HRMS (ESI) (Da): m/z calcd. 330.50789 for [M-3TFA]³⁺, found 330.50796.

### Example 2: Photophysical studies

Photophysical studies of synthesized compounds, details are summarized in the table below. Stock solution of each compound (final concentration 1 mg/mL) was prepared in DMSO (UV spectroscopy grade).

| **Medium** | **NIR dye** | **Abs λₘₐₓ (nm) ε (M⁻¹.cm⁻¹)** | **Em. λₘₐₓ (nm)** | **Lifetime (Ex. @ 670 nm, NanoLED)** | **Φ_{F} (%)** |
|---|---|---|---|---|---|
| **DMSO** | Aza-1 | 718 (78 600) | 739 | 1.9 ns (λₑₘ = 735 nm) | 6% |
| | Waza-1 | 720 (88 750) | 739 | 3 ns (λₑₘ = 735 nm) | 5% |
| | Waza-2 | 722 (115 000) | 740 | 3.2 ns (λₑₘ = 740 nm) | 26% |
| | Waza-BC1 | 724 (108 300) | 740 | 2.9 ns (λₑₘ = 740 nm) | 20% |
| **PBS (100 mM phosphate, 150 mM NaCl, pH 7.5)** | Aza-1 | 728 (31 650) | 721 | - | - |
| | Waza-1 | 709 (41 750) | 723 | 1.1 ns (λₑₘ = 735 nm) | 5% |
| | Waza-2 | 716 (43 000) | 725 | 1.3 ns (λₑₘ = 725 nm) | 7% |
| | Waza-BC1 | 715 (105 800) | 734 | 1.5 ns (λₑₘ = 740 nm) | 10% |
| **PBS + BSA (5%, w/v)** | Waza-2 | - | - | 2.9 ns (λₑₘ = 725 nm) | - |
| | Waza-BC1 | 715 (105 800) | 733 | 2 ns (λₑₘ = 740 nm) | 10% |
| **PBS + Serum (5%, w/v)** | Waza-BC1 | 715 (105 800) | - | - | 10% |

### pKa Determination of Aza-3, Aza-4, Aza-pH-1 and lifetime measurements:

pKa was determined using UV-Vis absorption measurements. The stock solution of each compound (final concentration = 1 mg/mL) was prepared in DMSO (UV spectroscopy grade), then diluted in the needed buffer solution (final concentration = 5 µM). Visible absorption spectrum (300-900 nm) of each solution was recorded at 25 °C. Variation of pH was done using different aq. buffer solutions (pH = 2, 0.1% TFA in ultrapure water; pH = 3.48 to pH = 5.68, aq. 0.1 M acetate buffer; pH = 5.9 to pH = 8.12, aq. 0.66 mM phosphate buffer; pH = 8.63 to 9.7, aq. 0.2 M borate buffer). In the three different cases ; i.e. for Aza-3, Aza-4, and Aza-pH-1, upon basification of the solution the absorption band at 650 nm vanishes and a new band centered at 700 nm appears. The absorbance at 700 nm was noted and plotted versus the buffer pH value. Curve fitting was achieved with OriginPro 8 software (fit sigmoidal, Boltzmann function) that also provided the inflection point whose x-coordinate corresponds to the pKa of phenol.

FLT was measured for the different compounds at different pH in order to show the influence of protonation/deprotonation state on this photophysical parameter.

| **Medium** | **NIR dye** | | **Abs λₘₐₓ (nm) ε (M⁻¹.cm⁻¹)** | | **Em λₘₐₓ (nm)** | **Lifetime (Ex. @ 670 nm, NanoLED)** | | **Φ_{F} (%)** |
|---|---|---|---|---|---|---|---|---|
| **DMSO + TFA (form OH)** | Aza-3 | | 697 (41 000) | | - | 2.2 ns (λₑₘ = 690 nm) | | - |
| | Aza-4 | | 689 (48 000) | | - | 2.5 ns (λₑₘ = 690 nm) | | - |
| | Aza-pH-1 | | 690 (50 500) | | 721 | 2.7 ns (λₑₘ = 690 nm) | | 23% |
| **DMEN+ Serum (10%, v/v) pH 2** | Aza-pH-1 | | 672 (41 000) | | 702 | - | | 4% |
| | | | | | | | | |
| | | Aza-3 | | Aza-4 | | | Aza-pH-1 | |
| pKa | | 6.8 | | 4 | | | 3.8 | |

FLT of Aza-3, Aza-4, and Aza-pH-1 in aq. buffers at different pH.

| | pH = 2.0 | pH = 3.5 | pH = 3.9 | pH = 6.1 | pH=9 |
|---|---|---|---|---|---|
| Aza-3 | | 0.63 ns (λₑₘ = 705 nm) | | 0.56 ns (λₑₘ = 705 nm) | non fluorescent |
| Aza-4 | 0.2 ns (λₑₘ = 690 nm) | | 0.19 ns (λₑₘ = 690 nm) | non fluorescent | non fluorescent |
| Aza-pH-1 | 0.19 ns (λₑₘ = 690 nm) | | 0.19 ns (λₑₘ = 690 nm) | non fluorescent | non fluorescent |

### Example 3: Bioconjugation on whole antibodies

Deglycosylation of antibodies: Antibody (2 mg/mL) in PBS pH 7.4 was incubated with 500 U/mg (one unit is defined as the amount of enzyme required to remove > 95% of the carbohydrate from 10 µg of denatured RNase B in 1 hour at 37°C in a total reaction volume of 10 µL) of protein of N-glycosidase F (PNGase F from NEB, New England Biolabs) overnight at 37 °C. The enzyme was then removed and the deglycosylated antibody was concentrated by centrifugation (Amicon Ultra-2 Centrifugal Filter Unit MWCO 50 kDa from Merck, three cycles of centrifugation at 4000 rpm for 15 min). The reaction was checked by LC-MS. Recovery yield: 90%.

Site-specific conjugation: deglycosylated antibody (4 mg/mL) in PBS was incubated with 20 equivalents of Waza-BC1 (30 mM in DMSO) and with 3 U/mg of antibody of microbial transglutaminase (MTGase from Zedira). The resulting solution was stirred overnight (16 h) in a thermomixer (900 rpm) at 37 °C. The reaction was checked by LC-MS. Excess of probes and MTGase were removed by FPLC purification. The degree of labeling (DOL) was calculated based on LC-MS analysis.

Random conjugation: bioconjugation was optimized to get a degree of labeling (DOL) around two for comparison purpose with site specific method. On this account, 7 equivalents of Waza-BC1-Sq at 15 mM (DMSO) was added to a solution of native antibody (2 mg/mL ) in 0.2 M bicarbonate buffer pH 9.2.The reaction mixture was then stirred in a thermomixer (900 rpm) for 6 h at 37 °C. The free probe was removed by FPLC. The degree of labeling (DOL) was calculated based on MALDI-TOF analysis.

Fast protein liquid chromatography (FPLC) purification: FPLC purification of the conjugates was performed on an ÄKTA^{™} 25 M system (GE Healthcare Life Sciences) with a Hitrap Mabselect^{™} column (MabSelect resin, Protein A, cross-linked agarose, column I.D. 7 mm, bed dimensions 7 x 25 mm, bed volume 1 mL). After deposition of the product, the conjugate was washed with 5 CV of PBS pH 7.4 (1 mL/min) followed by 5 CV of PBS 0.1 %Tween20 (1 mL/min) and 5 CV of PBS pH 7.4 (1 mL/min). The conjugate was then eluted with 25 mM acetic acid (1 mL/min) and collected. Purification was monitored at 280 and 700 nm. Thereafter, the solution was transferred to an ultra-centrifugal filter device (Amicon Ultra 2 mL, Ultracel cut-off 50 kDa from Merck Millipore) and centrifuged at 4000 rpm for 3x15 min in order to condition the mixture in PBS (pH 7.4). Purification monitoring was performed using UNICORN^{™} 7.2 interface software (GE Healthcare).

The recovered yield and masses of antibody engaged in each reaction are listed in the table bellowed with the corresponding DOL determined thanks to LC-ESI-MS analysis (for site-specific conjugation) or MALDI-TOF analysis (for random conjugation) after purification. LC-ESI-MS: Site-specific bioconjugation reactions were followed by reverse phase HPLC (Vanquish, Thermo Fisher Scientific) coupled to a high-resolution Orbitrap mass spectrometer (Exploris 240, Thermo Fisher Scientific) using an ESI source (positive mode). MALDI-TOF mass spectrometry: Analysis of random conjugates were performed by matrix-assisted laser desorption ionization mass spectrometry on a MALDI-ToF Microflex LRF instrument (Bruker). Samples were prepared with a saturated matrix of sinapinic acid: 3,5-dimethoxy-4-hydroxycinnamic acid (Sigma Aldrich), in a 4/6 water/acetonitrile mixture (H2O + 0.1% TFA/CH₃CN + 0.1% TFA). ). Degree of labeling (DOL) ratios were determined by comparison of native and conjugated antibody masses, using mMass 5.5.0 software. For more accurate DOL calculation (neglecting local signal variations) a Gaussian smoothing was applied (5 cycles, size 800 m/z).

| **Bioconjugates** | **Antibody used** | **Mass of antibody engaged** | **DOL** | **Bioconjugation Yield** |
|---|---|---|---|---|
| **Trastu-rd** | Trastuzumab | 2 mg | 1.9 | 65 % |
| **Trastu-ss** | Trastuzumab | 2 mg | 2.0 | 55 % |
| **Cetux-rd** | Cetuximab | 1.75 mg | 2.1 | 64 % |
| **Cetux-ss** | Cetuximab | 1.2 mg | 2.0 | 60% |

Results of the bioconjugation reactions

## Claims

1. A compound of following formula (I): 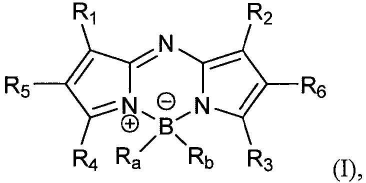 wherein:
R₁, R₂, R₃ and R₄, identical or different, represent a C₅-C₇ aryl or heteroaryl group, optionally substituted with at least one group chosen from halogen, nitro, -NR_{c}R_{d}, -OR_{d}, hydrazine, - CF₃, -CN, and groups constituted by or comprising a linker L and at least one group V,
R₅ and R₆, identical or different, represent a hydrogen, a halogen, a linear or branched (C₁-C₁₅)-group comprising an aldehyde, ketone, carboxylic acid or ester function, a nitrile, a sulfonate, a vinyl group optionally substituted by a ketone, ester or aromatic group, an imine substituted by an alkyl or aromatic group, an alkyne group optionally substituted by an alkyl or aromatic group, SPh, an aromatic chalcogen (SePh, TePh), an amide, a C₃-C₇ aryl or
heteroaryl group, optionally substituted by at least one group chosen from a halogen, - NR_{c}R_{d}, -OR_{d}, hydrazine, -CF₃ and -CN,
optionally R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN,
R_{c} and R_{d}, identical or different, represent hydrogen or a linear or branched (C₁-C₅₀)-alkyl group, optionally interrupted by at least one atom or group chosen from -O-, -NR-, -S-, - C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, optionally substituted by at least one group comprising one or more heteroatoms chosen from O, N, P and/or S, in particular a group chosen from quaternary ammonium, sulphate, sulfonate, and phosphonate,
Rₐ and R_{b}, identical or different, represent:
- a linear or branched (C₁-C₅₀)-alkyl group, optionally interrupted by at least one atom or group chosen from -O-, -NR-, -N⁺RR'-, -S-, -C(=O)-, -O-C(=O)-, -C(=O)-O-, - NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C≡C-, arene diyl, in particular benzene diyl, and heteroarene diyl,
- a group constituted by or comprising a linker L and at least one group V,
V is chosen from:
- groups comprising one or more heteroatoms chosen from O, N, P and/or S, in particular a group chosen from quaternary ammonium, sulphate, sulfonate, and phosphonate,
- bioconjugable groups, in particular chosen from amine, ammonium, carboxylic acid, activated esters, in particular N-hydrosuccinimidyl, N-hydroxysulfosuccinimide, pentafluorophenyl, tetrafluorophenyl, squarate, in particular diethylsquarate, maleimide, thiol, isothiocyanate, isocyanate, oxadiazolyl methyl sulfone, azoture, optionally substituted tetrazine, triazole, alkyne, in particular terminal alkyne, trans-cyclooctene, cyclooctyne, in particular dibenzocyclooctyne and bicyclononyne,
- a biological vector, in particular a cyclic or linear peptide, an antibody, an antibody fragment, a nanobody, an affibody, an aptamer, a short DNA or RNA sequence, a polysaccharide, a lipid, a sugar, an amino acid, a vitamin, a AMD3100 or AMD3100-type molecule, a prostate-specific membrane antigen (PSMA) ligand, a steroid, a fatty acid, a polyamine, a polyphenol, a DNA base or a caffeine derivative,
- a nanoparticle, in particular a nanoparticle of 10 kDa or less or lipidic nanoparticles, more particularly a gold nanocluster or a lipid nanoparticle, for example a lipidot,
- a metal complex, in particular for therapeutic purposes, formed by a chelating agent and a metal,
- a radiometallic complex, formed by a chelating agent, for example DOTA (1,4,7,10-tetraazacyclododecane-1,4,7,10-tetraacetic acid), and a radiometal, in particular for therapeutic or diagnostic purposes,
- a metal or radiometal chelating agent, for example DOTA,
L is in particular a linear or branched (C₁-C₅₀)-alkyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl,
R and R', identical or different, represent hydrogen or a linear or branched (C₁-C₆)-alkyl group,
or one of its tautomers,
with the proviso that:
- at least one of R₃ and R₄, in particular R₃ and R₄, represent(s) a C₅-C₇ heteroaryl group, in particular a thiophenyl group, optionally substituted with at least one group chosen from halogen, -NR_{c}R_{d}, -OR_{d}, hydrazine, -CF₃, -CN, and groups constituted by or comprising a linker L and at least one group V, with, in this case, R₁ and/or R₂ being different from *p*-N(Me)₂-phenyl, and optionally R^{a} and/or R^{b} being further different from -C≡C-CH₂-NH(CH₃)₂ and/or -C≡C-CH₂-N⁺(CH₃)₃, in particular different from -C≡C-CH₂-NH(CH₃)₂;
- at least one of R^{a} and R^{b}, represents a group V' of following formula -Lₐ-X-L_{b}-V, wherein Lₐ and L_{b}, identical or different, represent a linear or branched (C₁-C₅₀)-alkyl group, optionally interrupted by at least one atom or group chosen from -O-, - S-, -C(R)=C(R')-, -C=C-, X represents -NR- or -N⁺RR'-, and V is as defined above; and/or
- at least one of R₁, R₂, R₃ and R₄, in particular one of R₁, R₂, R₃ and R₄, represents a C₅-C₇ aryl or heteroaryl group, substituted with at least one group chosen from groups constituted by or comprising a linker L and at least one group V; and/or
- R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and - CN.

2. The compound according to claim 1, wherein R₅ and/or R₆, in particular R₅ and R₆, represent a hydrogen.

3. The compound according to anyone of the preceding claims, wherein R₁ and/or R₂, in particular R₁ and R₂, represent a C₅-C₇ aryl, optionally substituted with at least one group chosen from halogen, nitro, -NR_{c}R_{d}, -OR_{d}, hydrazine, -CF₃, and -CN.

4. The compound according to anyone of the preceding claims, wherein:
- R₃ and/or R₄, in particular R₃ and R₄, represent a heteroaryl group, optionally substituted with at least one group chosen from halogen, nitro, -NR_{c}R_{d}, -OR_{d}, hydrazine, -CF₃, and - CN; or
- R₃ and/or R₄, in particular R₃ or R₄, represent a C₅-C₇ aryl or heteroaryl group, substituted with at least one group constituted by or comprising a linker L and at least one group V.

5. The compound according to anyone of the preceding claims, wherein at least one of R^{a} and R^{b}, in particular R^{a} or R^{a} and R^{b}, represent(s) a group constituted by or comprising a linker L and at least one group V.

6. The compound according to anyone of the preceding claims, wherein at least one of R^{a} and R^{b}, in particular R^{a} or R^{a} and R^{b}, represent(s) a group V' of following formula -Lₐ-X-L_{b}-V, wherein Lₐ and L_{b}, identical or different, represent a linear or branched (C₁-C₅₀)-alkyl group, optionally interrupted by at least one atom or group chosen from -O-, -S-, - C(R)=C(R')-, -C=C-, X represents -NR- or -N⁺RR'-, and V is as defined above.

7. The compound according to anyone of the preceding claims, wherein Rₐ and/or R_{b}, in particular R^{a} and R_{b}, represent(s) a group of formula -C≡C-CH₂-NR-L₆-V or -C≡C-CH₂-N⁺RR'-L₆-V, wherein L_{b}, R, R', and V are as defined in claim 1, being in particular -C ≡C-CH₂-NMe-L_{b}-V or -C≡C-CH₂-N⁺(Me)₂-L_{b}-V.

8. The compound according to claim 1, chosen from: 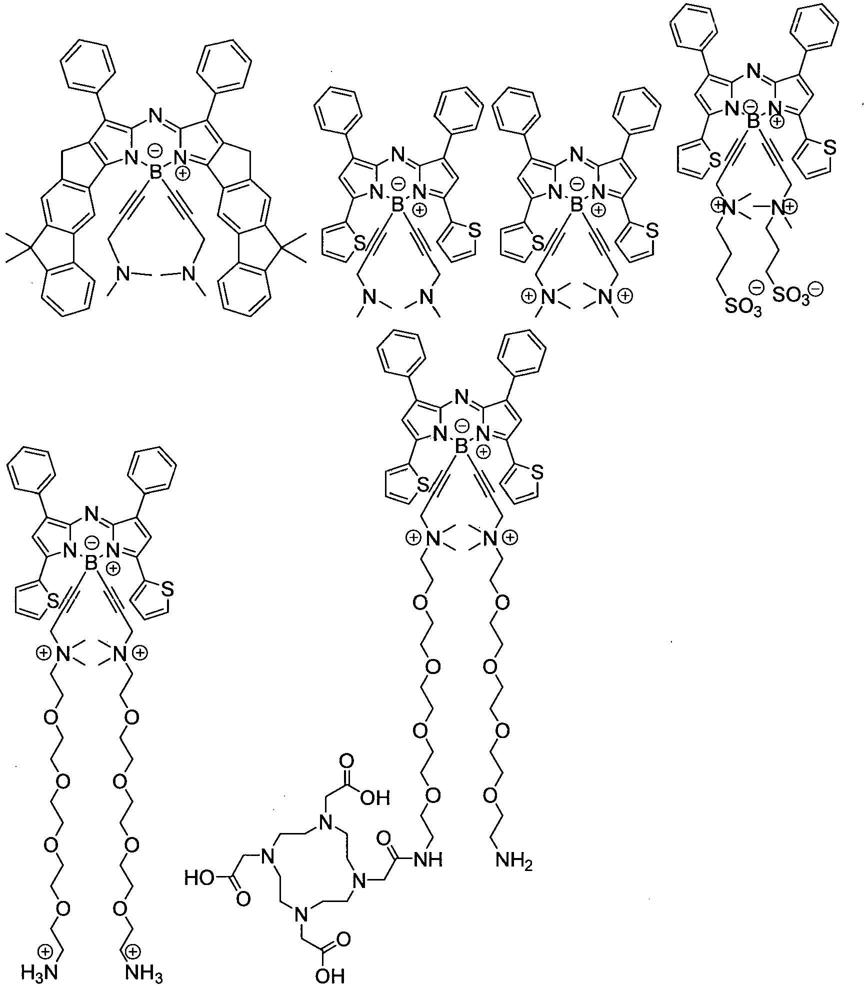 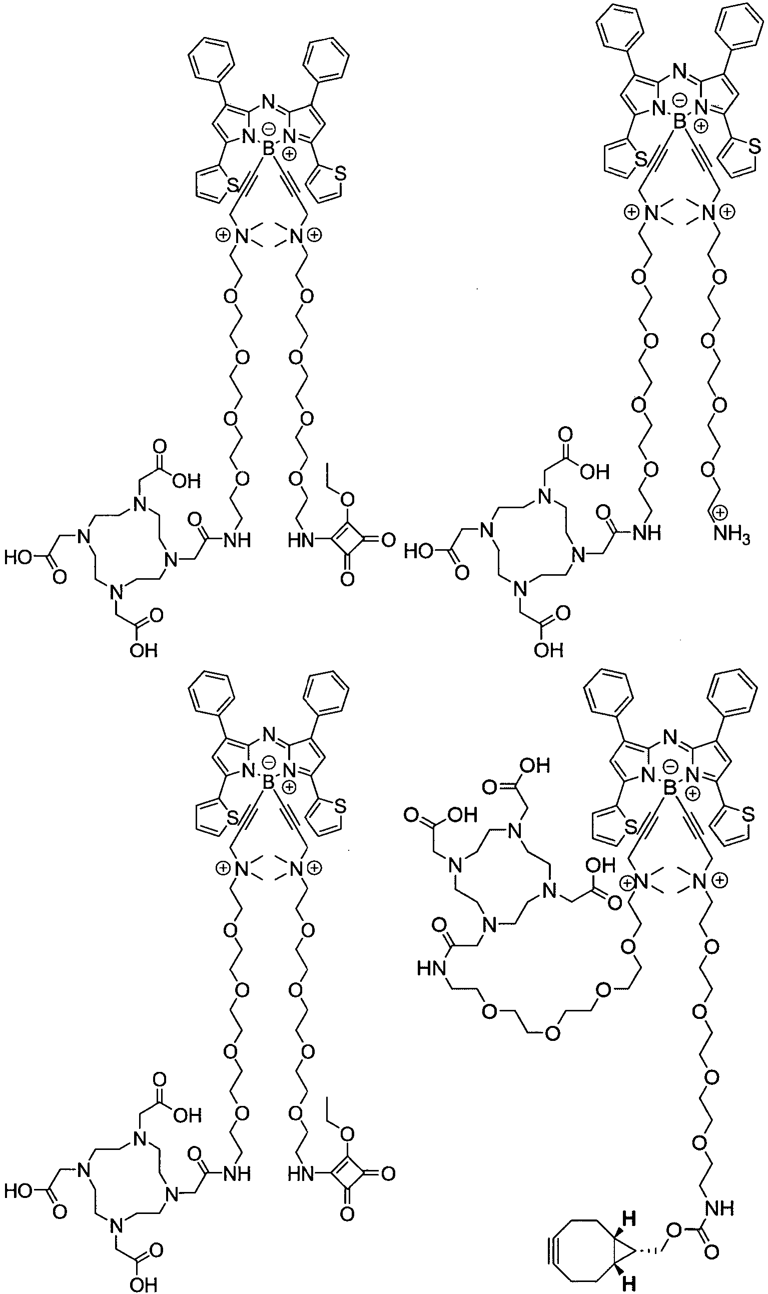 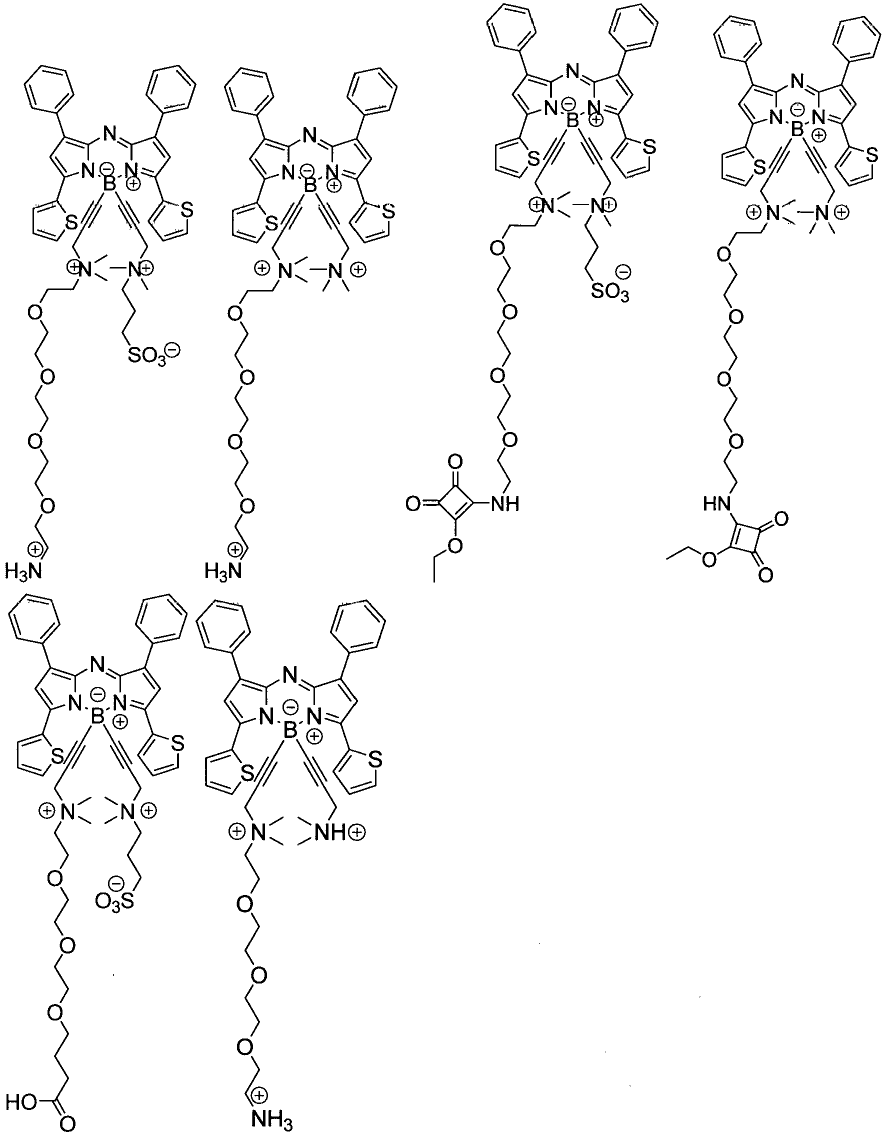 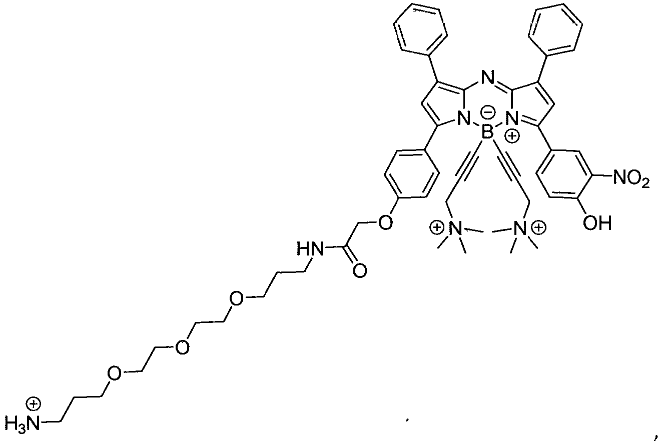 said compounds being optionally, if applicable, in the form of a formate or trifluoroacetate salt.

9. A compound of following formula (II), for use in a method of *in vivo* fluorescence-lifetime imaging: 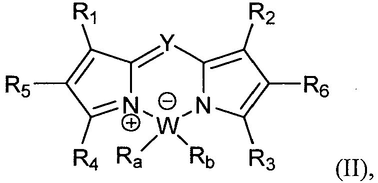 wherein:
W is chosen from B, PO₂, Ga, Al, In, Ru, Fe, Pt, Pd, Zr, Zn, Cu, Bi, Sb, Au, Rh, Ge, Sn,
Ti;W being in particular B;
Y is N or CZ, Y being in particular N;
Z is chosen from:
- H;
- Halogens;
- OR" and SR", wherein R" represents hydrogen or a linear or branched (C₁-C₆)-alkyl group;
- linear or branched (C₁-C₆)-alkyl group;
- a C₅-C₇ aryl or heteroaryl group being optionally substituted with at least one group chosen from halogen, nitro, -NR_{c}R_{d}, -OR_{d}, hydrazine, -CF₃, -CN, and groups constituted by or comprising a linker L and at least one group V,
R₁, R₂, R₃ and R₄, identical or different, represent a linear or branched (C₁-C₆)-alkyl group, a C₅-C₇ aryl or heteroaryl group, a -CH₂=CH₂-(C₅-C₇) aryl or -CH₂=CH₂-(C₅-C₇) heteroaryl group, said aryl or heteroaryl group being optionally substituted with at least one group chosen from halogen, nitro, -NR_{c}R_{d}, -OR_{d}, hydrazine, -CF₃, -CN, and groups constituted by or comprising a linker L and at least one group V,
R₅ and R₆, identical or different, represent a hydrogen, a halogen, a linear or branched (C₁-C₁₅)-group comprising an aldehyde, ketone, carboxylic acid or ester function, a nitrile, a sulfonate, a vinyl group optionally substituted by a ketone, ester or aromatic group, an imine substituted by an alkyl or aromatic group, an alkyne group optionally substituted by an alkyl or aromatic group, SPh, an aromatic chalcogen (SePh, TePh), an amide, a C₃-C₇ aryl or heteroaryl group, optionally substituted by at least one group chosen from a halogen, - NR_{c}R_{d}, -OR_{d}, hydrazine, -CF₃ and -CN,
optionally R₄ and R₅ and/or R₃ and R₆ are covalently bonded and together form a C₅-C₇ aryl or heteroaryl group, or a polycyclic aromatic group, optionally substituted by at least one group chosen from (C₁-C₆)-alkyl, halogen, -NR_{c}R_{d}, -OR_{d}, hydrazine, -CF₃ and -CN,
R_{c} and R^{d}, identical or different, represent hydrogen or a linear or branched (C₁-C₅₀)-alkyl group, optionally interrupted by at least one atom or group chosen from -O-, -NR-, -S-, - C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl, optionally substituted by at least one group comprising one or more heteroatoms chosen from O, N, P and/or S, in particular a group chosen from quaternary ammonium, sulphate, sulfonate, and phosphonate,
Rₐ and R_{b}, identical or different, are absent or represent:
- a halogen, in particular -F or -Cl,
- a linear or branched (C₁-C₅₀)-alkyl group, optionally interrupted by at least one atom or group chosen from -O-, -NR-, -N⁺RR'-, -S-, -C(=O)-, -O-C(=O)-, -C(=O)-O-, - NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl,
- a group constituted by or comprising a linker L and at least one group V,
Rₐ and R_{b} are absent when W is chosen from PO₂, Ga, Al, In, Ru, Fe, Pt, Pd, Zr, Zn, Cu, Bi, Sb, Au, Rh, Ge, Sn, Ti;
R^{a} and R_{b} are present when W is B;
V is chosen from:
- groups comprising one or more heteroatoms chosen from O, N, P and/or S, in particular a group chosen from quaternary ammonium, sulphate, sulfonate, and phosphonate,
- bioconjugable groups, in particular chosen from amine, ammonium, carboxylic acid, activated esters, in particular N-hydrosuccinimidyl, N-hydroxysulfosuccinimide, pentafluorophenyl, tetrafluorophenyl, squarate, in particular diethylsquarate, maleimide, thiol, isothiocyanate, isocyanate, oxadiazolyl methyl sulfone, azoture, optionally substituted tetrazine, triazole, alkyne, in particular terminal alkyne, trans-cyclooctene, cyclooctyne, in particular dibenzocyclooctyne and bicyclononyne,
- a biological vector, in particular a cyclic or linear peptide, an antibody, an antibody fragment, a nanobody, an affibody, an aptamer, a short DNA or RNA sequence, a polysaccharide, a lipid, a sugar, an amino acid, a vitamin, a AMD3100 or AMD3100-type molecule, a prostate-specific membrane antigen (PSMA) ligand, a steroid, a fatty acid, a polyamine, a polyphenol, a DNA base or a caffeine derivative,
- a nanoparticle, in particular a nanoparticle of 10 kDa or less or lipidic nanoparticles, more particularly a gold nanocluster or a lipid nanoparticle, for example a lipidot,
- a metal complex, in particular for therapeutic purposes, formed by a chelating agent and a metal,
- a radiometallic complex, formed by a chelating agent, for example DOTA (1,4,7,10-tetraazacyclododecane-1,4,7,10-tetraacetic acid), and a radiometal, in particular for therapeutic or diagnostic purposes,
- a metal or radiometal chelating agent, for example DOTA,
L is in particular a linear or branched (C₁-C₅₀)-alkyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-, -C(R)=C(R')-, -C=C-, arene diyl, in particular benzene diyl, and heteroarene diyl,
R and R', identical or different, represent hydrogen or a linear or branched (C₁-C₆)-alkyl group,
or one of its tautomers.

10. The compound for use according to claim 9, wherein said compound emits at a wavelength greater than or equal to 650 nm, in particular greater than or equal to 700 nm.

11. The compound for use according to claim 9 or 10, wherein said use includes a step of measuring fluorescence variations linked to variations in physiological conditions chosen from pH, oxygen content, presence of enzymes, polarity and viscosity of cell organelles.

12. The compound for use according to anyone of claims 9 to 11, wherein said compound is chosen from:
